# EUROPEAN PATENT APPLICATION

(11) **EP 1 300 142 A1**
(43) Date of publication of application: **09.04.2003**
(21) Application number: 01945707.6
(22) Date of filing: 29.06.2001
(51) Int. Cl.: A61K 31/18, A61K 31/44, A61K 31/341, A61K 31/381, A61K 45/00, C07C 311/51, C07D 213/81, C07D 213/82, C07D 307/68, C07D 333/40, A61P 3/10

(54) **HYPOGLYCEMICS**

(30) Priority: 05.07.2000 JP 2000203993; 28.07.2000 JP 2000229505
(71) Applicant: Ajinomoto Co., Inc., Tokyo 104-0031 (JP)
(72) Inventor: IKAWA, Hiroshi, Setagaya-Ku, Tokyo 156-0051 (JP); NISHIMURA, Masato, FUJIREBIO INC., Tokyo 103-0007 (JP); OKADA, Keiji, FUJIREBIO INC., Tokyo 103-0007 (JP); NAKAMURA, Takashi, FUJIREBIO INC., Tokyo 103-0007 (JP); INABA, Jiro, Tama-Shi, Tokyo 206-0031 (JP); KOJIMA, Kazuhiro, FUJIREBIO INC., Tokyo 103-0007 (JP); KOSONO, Hideki, Kamakura-Shi, Kanagawa 248-0031 (JP)
(74) Representative: Nicholls, Kathryn Margaret
(86) International application number: JP0105625
(87) International publication number: WO02002101

(57) **Abstract**

The present invention provides a hypoglycemic agent containing an acylsulfonamide derivative such as a compound of the following formula or an analog thereof, which agent is usable for preventing and curing diabetes:

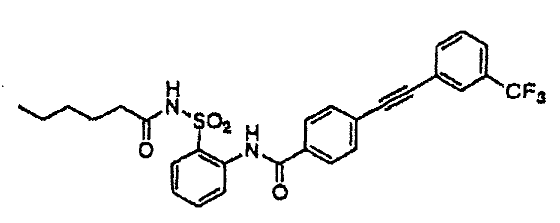

## Description

### Background of the Invention

The present invention relates a hypoglycemic agent having a hypoglycemic effect and also an activity of inhibiting acetyl CoA carboxylase (hereinafter referred to ACC).

Diabetes is a diseases caused by various factors such as overeating, lack of exercise, stress and hereditary factors. The number of the patients suffering from diabetes is increasing as the life of the people is improved. At present, the number of diabetes cases is so large that this disease is called "a national disease" in Japan. Diabetes is classified into two types, i. e. insulin dependent diabetes mellitus (IDDM) and non-insulin dependent diabetes mellitus (NIDDM). In Japan, at least 90 % of diabetes cases is NIDDM. Patients suffering from NIDDM scarcely have the subjective symptoms and when the patients are found to suffer from this disease, the disease has already progressed in many cases. In such a case, a suitable therapy is required for avoiding complications.

For the treatment of NIDDM, dietetic therapy or kinetotherapy is employed at first. By such a therapy, the effects such as reduction in obesity, increase in the insulin sensitivity and reduction of insulin requirement in peripheries and reduction of endogenous insulin requirement can be expected. As a result, the blood glucose level can be controlled. However, in many cases, a sufficient effect in reducing the blood glucose level cannot be obtained by the dietetic therapy or kinetotherapy. In such cases, the patients are treated with medicines.

Hypoglycemic agents known at present include insulin preparations, insulin secretion accelerators, α-glycosidase inhibitors, biguanide and glitazone compounds. Depending on the pathological conditions of the patients, a medicine is given alone or in combination with other medicines having different action mechanisms.

In the background of NIDDM onset, it is considered to be important to solve the obesity caused by excess *in vivo* energy due to overeating and lack of exercise and also insulin resistance induced thereby. In the above-described medicines, glitazone compounds, capable of reducing the blood glucose level by releasing the insulin resistance, attract the greatest attention.

Glitazone compounds induce the differentiation of fat cells by activating PPARγ which is one of intranuclear receptors, and thus improve insulin resistance of peripheral tissues and exhibit the pharmaceutical effect. However, these compounds have strong side effects, and a serious hepatopathy including cases of death was reported. Further, because these compounds accelerate the differentiation of fat cells, the fat accumulation is accelerated to induce the obesity. Also in clinical cases, reduction or weakening of the pharmaceutical effect of them by the increase in the body weight or accumulation of fats was also reported.

### Disclosure of the Invention

The treatment with the above-described hypoglycemic agents is not yet satisfactory and, in addition, the glitazone compounds which attract the attention are also not yet perfect because of the above-described side effects. Under these circumstances, an object of the present invention is to develop a new hypoglycemic agent having a hypoglycemic effect equal to or superior to the effects of the conventional medicines and free from the side effects unlike the glitazone compounds.

After intensive investigations made for the purpose of solving the problems, the inventors have found a new hypoglycemic agent and also a new agent having the hypoglycemic effect and also acetyl CoA carboxylase-inhibiting activity. The present invention has been completed on the basis of this finding.

The detailed description will be made on the present invention.

ACC is an enzyme catalyzing the synthesis of malonyl CoA from acetyl CoA. ACC is a rate limiting enzyme in the biosynthesis of long-chain fatty acids. It is known that malonyl CoA synthesized from acetyl CoA with ACC controls carnitine acyltransferase concerning the oxidation of free long-chain fatty acids as the energy sources. Further, it is considered that ACC activation participates in the activation in the synthesis of fatty acids in visceral adipose tissues.

Therefore, when ACC activity is inhibited, the biosynthesis of long-chain fatty acids *in vivo* is also suppressed and the metabolism is accelerated to reduce the amount of long-chain fatty acids *in vivo* and, as a result, the biosynthesis of triglycerides is controlled.

Thus, the hypoglycemic agent of the present invention is very effective in preventing and treating diabetes because of its direct effect of reducing the blood glucose of diabetes patients and also indirect effect of controlling the accumulation of visceral fats by the ACC inhibition effect.

An example of the hypoglycemic agents and also hypoglycemic agents further having ACC inhibiting activity is acylsulfonamide derivatives of the following general formula (I): 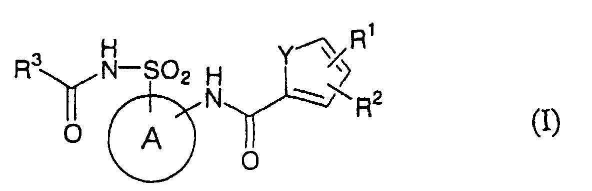 wherein R¹ represents a substituted or unsubstituted C₁ to C₁₂ alkyl group, a substituted or unsubstituted C₂ to C₁₂ alkenyl group, a substituted or unsubstituted C₂ to C₁₂ alkynyl group or a substituted or unsubstituted C₁ to C₁₂ alkoxyl group, R² represents hydrogen atom, hydroxyl group, mercapto group, a substituted or unsubstituted C₁ to C₆ alkoxyl group, a substituted or unsubstituted C₁ to C₆ alkylthio group, nitro group, a halogen atom, trichloromethyl group, trifluoromethyl group or cyano group, R³ represents a substituted or unsubstituted C₁ to C₂₀ alkyl group, a substituted or unsubstituted C₂ to C₂₀ alkenyl group, a substituted or unsubstituted C₂ to C₂₀ alkynyl group, a substituted or unsubstituted aromatic hydrocarbon group, a substituted or unsubstituted aromatic heterocyclic group, a substituted amino group, a substituted or unsubstituted C₁ to C₂₀ alkoxyl group, a substituted or unsubstituted C₂ to C₂₀ alkenyloxyl group, a substituted or unsubstituted C₂ to C₂₀ alkynyloxyl group or a group of the formula: R⁴O-, wherein R⁴ represents a substituted or unsubstituted aromatic hydrocarbon group or a substituted or unsubstituted aromatic heterocyclic group, Y represents a group of the formula: -CH=CH-, -N=CH- or -CH=N-, sulfur atom or oxygen atom, and ring A represents a substituted or unsubstituted aromatic hydrocarbon group, a substituted or unsubstituted aromatic heterocyclic group or a substituted or unsubstituted cycloalkyl group.

The present invention also provides a hypoglycemic agent containing a compound(s) having an activity of inhibiting acetyl CoA carboxylase as an active ingredient(s).

The present invention also provides a hypoglycemic agent having an activity of inhibiting acetyl CoA carboxylase.

The present invention also provides an agent for improving insulin resistance, which contains a compound having an activity of inhibiting acetyl CoA carboxylase as an active ingredient.

The active ingredients include the acylsulfonamide derivatives of the general formula (I).

The present invention also provides an acetyl CoA carboxylase inhibitor containing an acylsulfonamide derivative of general formula (I) as an active ingredient.

### Description of the Preferred Embodiments

When the hypoglycemic agent containing the acylsulfonamide derivative of above general formula (I) as the active ingredient is administered to human beings, the effective dose thereof varies depending on the symptoms and age of the patient. For example, 5 to 30 mg/day of the active ingredient is preferably orally administered once or dividedly into three portions a day.

The hypoglycemic agent of the present invention can be orally administered in various dosage forms such as tablets, capsules, granules, a powder, troches and a liquid. These preparations can be produced by known methods. For example, an acylsulfonamide derivative of the general formula (I) is suitably combined with a filler such as starch, mannitol or lactose; a binder such as sodium carboxymethylcellulose or hydroxypropylcellulose; a disintegrator such as crystalline cellulose or carboxymethylcellulose; a lubricant such as talc or magnesium stearate; and/or a fluidizing agent such as light anhydrous silicic acid to form tablets, capsules, granules, a powder, troches, etc.

The hypoglycemic agent of the present invention can be in the form of an injection. The injection can be prepared by previously dispersing or dissolving the hypoglycemic agent in an aqueous carrier such as physiological saline with a surfactant or a dispersing agent, or the hypoglycemic agent can be kept in the form of a crystalline preparation for injection or freeze-dried preparation which can be dispersed or dissolved each time for the injection. The aqueous carrier may contain a pH regulator or a stabilizing agent, if necessary.

The dose and route of administration of the injection are not particularly limited. A safe and necessary amount of the injection can be given by the intravenous, arterial, subcutaneous or intraperitoneal injection at once or intravenous drip infusion.

As will be apparent from the results obtained in Examples described below, the acylsulfonamide derivatives of the above general formula (I) provided by the present invention have an excellent hypoglycemic effect and they are very useful for the treatment of diabetes cases. Further, because they also have ACC inhibiting activity, they are capable of decreasing long-chain fatty acids *in vivo* and, as a result, they control the biosynthesis of triglycerides to inhibit obesity before and after diabetes.

The detailed description will be made on the acylsulfonamide derivatives of general formula (I). In this specification, the term "C₁ to C₁₂ alkyl groups" include linear, branched and cyclic groups such as methyl, ethyl, n-propyl, 1-methylethyl, cyclopropyl, n-butyl, 2-methylpropyl, 1-methylproyl, 1,1-dimethylethyl, cyclobutyl, n-pentyl, 1-methylbutyl, 2-methylbutyl, 3-methylbutyl, cyclopentyl, 2,2-dimethylpropyl, n-hexyl, 1-methylpentyl, 2-methylpentyl, 4-methylpentyl, 1-ethylbutyl, 2-ethylbutyl, 3,3-dimethylbutyl, cyclohexyl, n-heptyl, 1-methylhexyl, 2-methylhexyl, 5-methylhexyl, 4,4-dimethylpentyl, 1-propylbutyl, 2-ethylpentyl, cyclohexylmethyl, 1,1-diethylpropyl, cycloheptyl, n-octyl, 1-methylheptyl, 6-methylheptyl, 1-ethylhexyl, 2-ethylhexyl, 2-cyclohexylethyl, 5,5-dimethylhexyl, cyclooctyl, n-nonyl, 1-methyloctyl, 7-methyloctyl, 6,6-dimethylheptyl, n-decyl, 1-methylnonyl, 8-methylnonyl, 7,7-dimethyloctyl, n-undecyl, 1-methyldecyl, 9-methyldecyl, 8,8-dimethylnonyl, n-dodecyl, 1-methylundecyl, 10-methylundecyl, 5-methylundecyl and 9,9-dimethyldecyl groups. These alkyl groups may have various substituents. Examples of the substituents include halogen atoms such as chlorine, bromine, iodine and fluorine atoms, nitro group, amino group, cyano group, oxo group, hydroxyl group, alkoxyl groups, thiol group, trichloromethyl group, trifluoromethyl group, aromatic hydrocarbon groups such as phenyl and naphthyl groups, and aromatic heterocyclic groups such as thienyl, furyl and pyridyl groups. These aromatic hydrocarbon groups and aromatic heterocyclic groups may further have substituents such as halogen atoms, alkyl groups, alkoxyl groups, nitro group, amino group, cyano group, hydroxyl group and thiol group.

The term "C₁ to C₂₀ alkyl groups" may also include linear, branched and cyclic groups. They include the above-described "C₁ to C₁₂ alkyl groups" and, in addition, dodecyl, tetradecyl, pentadecyl, hexadecyl, heptadecyl, octadecyl, nonadecyl and eicosyl groups. These alkyl groups may have various substituents. Examples of them may be the same as those listed above as the substituents of the C₁ to C₁₂ alkyl groups.

The term "substituted or unsubstituted aromatic hydrocarbon groups" indicates monocyclic or polycyclic aromatic hydrocarbon groups which may have one or more various substituents on the ring. They iclude, for example, phenyl, methylphenyl, dimethylphenyl, methoxyphenyl, dimethoxyphenyl, nitrophenyl, dinitrophenyl, chlorophenyl, dichlorophenyl, bromophenyl, dibromophenyl, iodophenyl, fluorophenyl, trifluoromethylphenyl, aminophenyl, hydroxyphenyl, mercaptophenyl, α-naphthyl and β-naphthyl groups.

The term "substituted or unsubstituted aromatic heterocyclic groups" indicates those having a five-membered ring or six-membered ring containing at least one hetero atom such as nitrogen atom, sulfur atom or oxygen atom. These groups may be condensed with benzene ring and have one or more substituents on the ring. They include, for example, pyridyl, furyl, thienyl, indolyl, quinolyl, isoquinolyl, benzofuranyl, benzothienyl, imidazolyl, benzimidazolyl, thiazolyl, oxazolyl, pyrazolyl, pyrimidyl, pyrazinyl, isoxazolyl, isoindolyl and pyrrolyl groups.

The "C₂ to C₁₂ alkenyl groups" may be linear or branched groups. They include, for example, 1-methyl-1-propenyl, 1-methyl-2-propenyl, 2-methyl-2-propenyl, ethenyl, 1-methylethenyl, 1-propenyl, 2-propenyl, 1-butenyl, 2-butenyl, 2-pentenyl, 1-penenyl, 3-methylbutenyl, 1,3-butanedienyl, 1-hexenyl, 2-hexenyl, 3,3-dimethyl-1-butenyl, 4,4-dimethyl-1-pentenyl, 1,3-pentanedienyl, 1,3-hexanedienyl, 2-cyclohexylethenyl, octenyl, nonenyl, decenyl, undecenyl and dodecenyl groups. These alkenyl groups may have various substituents. Examples of them may be the same as those listed above as the substituents of the C₁ to C₁₂ alkyl groups.

The "C₂ to C₂₀ alkenyl groups" may be linear, branched or cyclic groups. They include, in addition to those described above as examples of "C₂ to C₁₂ alkyl groups", for example, tridecenyl, tridecadienyl, tetradecenyl, tetradecadienyl, pentadecenyl, pentadecadienyl, pentadecatrienyl, hexadecenyl, hexadecadienyl, hexadecatrienyl, heptadecenyl, heptadecadienyl, heptadecatrienyl, octadecenyl, octadecadienyl, octadecatrienyl, nonadecenyl, nonadecadienyl, nonadecatrienyl, eicosenyl, eicosadienyl and eicosatrienyl groups. These alkenyl groups may have various substituents. Examples of them may be the same as those listed above as the substituents of the C₁ to C₁₂ alkyl groups.

The "C₂ to C₁₂ alkynyl groups" may be linear or branched groups. They include, for example, 1-propynyl, 2-propynyl, 1-methyl-2-propynyl, 1-ethyl-2-propynyl, ethynyl, 1-butynyl, 2-butynyl, 1,3-butadiynyl, 1-pentynyl, 2-pentynyl, 1,3-pentadiynyl, 1-hexynyl, 2-hexynyl and 1,3-hexadiynyl groups. These alkynyl groups may have various substituents. Examples of them may be the same as those listed above as the substituents of the C₁ to C₁₂ alkyl groups. Other particularly preferred substituents include 1 or more fluorine atoms, trifluoromethyl groups and phenyl groups substituted with trifluoromethoxyl group.

The "C₂ to C₂₀ alkynyl groups" may be linear, branched or cyclic groups. They include, in addition to those described above as examples of "C₂ to C₁₂ alkyl groups", for example, tridecynyl, tridecadiynyl, tetradecynyl, tetradecadiynyl, pentadecynyl, pentadecadiynyl, pentadecatriynyl, hexadecynyl, hexadecadiynyl, hexadecatriynyl, heptadecynyl, heptadecadiynyl, heptadecatriynyl, octadecynyl, octadecadiynyl, octadecatriynyl, nonadecynyl, nonadecadiynyl, nonadecatriynyl, eicosynyl, eicosadiynyl and eicosatriynyl groups. These alkenyl groups may have various substituents. Examples of them may be the same as those listed above as the substituents of the C₁ to C₁₂ alkyl groups.

The "substituted amino groups" are amino groups wherein the nitrogen atom is substituted with one or two of the above-described, substituted or unsubstituted C₁ to C₂₀ alkyl groups, substituted or unsubstituted C₂ to C₂₀ alkenyl groups, substituted or unsubstituted C₂ to C₂₀ alkynyl groups, substituted or unsubstituted aromatic hydrocarbon groups or substituted or unsubstituted aromatic heterocyclic groups. The alkyl group may form a 5- to 7-membered saturated heterocycle which may contain nitrogen atom, oxygen atom or sulfur atom together with the nitrogen atom to which the alkyl group is bonded. The substituted amino groups include, for example, methylamino, ethylamino, propylamino, butylamino, pentylamino, hexylamino, dimethylamino, diethylamino, dipropylamino, 2-propenylamino, 2-butenylamino, 3-butenylamino, 1-pyrrolidinyl, piperidino, 1-piperazinyl, morpholino, thiomorpholino, perhydroazepinyl, phenylamino, naphthylamino, pyridylamino, furylamino and thienylamino groups.

The term "C₁ to C₁₂ alkoxyl groups" indicates alkyl-substituted oxy groups in which the alkyl groups are as defined above. Examples of these groups are methoxyl, ethoxyl, n-propoxyl, 1-methylethoxyl, n-butoxyl, 2-methylpropoxyl, 1-methylpropoxyl, 2-methyl-2-propoxyl, n-pentyloxyl, 3-methylbutoxyl, n-hexyloxyl, 4-methylpentoxyl, n-pentyloxyl, n-octyoxyl, n-nonyloxyl, n-decyloxyl and n-undecyloxyl groups. These alkyl groups may have various substituents. Examples of them may be the same as those listed above as the substituents of the C₁ to C₁₂ alkyl groups.

The "C₁ to C₂₀ alkoxyl groups" may be linear, branched or cyclic groups. They include, in addition to those described above as examples of "C₁ to C₁₂ alkoxyl groups", for example, tridecyloxyl, tetradecyloxyl, pentadecyloxyl, hexadecyloxyl, heptadecyloxyl, octadecyloxyl, nonadecyloxyl and eicosyloxyl groups. These alkoxyl groups may have various substituents. Examples of them may be the same as those listed above as the substituents of the C₁ to C₁₂ alkyl groups.

The term "C₁ to C₆ alkylthio groups" indicates alkyl-substituted thio groups wherein the alkyl groups are as defined above. Examples of them include methylthio, ethylthio, n-propylthio, 1-methylethylthio, n-butylthio, 2-methylpropylthio, 1-methylpropylthio, 2-methyl-2-propylthio, n-pentylthio, 3-methylbutylthio, n-hexylthio and 4-methylpentylthio groups. These alkylthio groups may have various substituents. Examples of them may be the same as those listed above as the substituents of the C₁ to C₁₂ alkyl groups.

The ring represented by A in the acylsulfonamide derivatives of the above general formula (I) provided by the present invention is the above-described aromatic hydrocarbon group or aromatic heterocyclic group. The ring A is particularly preferably phenyl group. As for the substitution mode of these groups, it is desirable that the acylsulfonamide side chain and the amide side chain have their substitution sites at the 1,2-position, or at the 1,1-position when A is a cycloalkyl group.

In the above general formula (I) of the acylsulfonamide derivative, R³ preferably represents a substituted or unsubstituted C₃ to C₈ alkyl group, a substituted or unsubstituted C₄ to C₈ alkenyl group, a substituted or unsubstituted C₄ to C₈ alkynyl group, a substituted or unsubstituted phenyl group, a substituted or unsubstituted naphthyl group, a substituted or unsubstituted C₃ to C₉ alkoxyl group, a substituted or unsubstituted C₄ to C₈ alkenyloxyl group, or a substituted or unsubstituted C₄ to C₈ alkynyloxyl group.

In the above general formula (I) of the acylsulfonamide derivative, R¹ preferably represents a C₁ to C₄ alkyl group having a substituted or unsubstituted aromatic hydrocarbon group or a substituted or unsubstituted aromatic heterocyclic group as the substituent, a C₂ to C₄ alkenyl group having a substituted or unsubstituted aromatic hydrocarbon group or a substituted or unsubstituted aromatic heterocyclic group as the substituent, a C₂ to C₄ alkynyl group having a substituted or unsubstituted aromatic hydrocarbon group or a substituted or unsubstituted aromatic heterocyclic group as the substituent, or C₁ to C₄ alkoxyl group having a substituted or unsubstituted aromatic hydrocarbon group or a substituted or unsubstituted aromatic heterocyclic group as the substituent.

In acylsulfonamide derivatives of the above general formula (I), R¹ preferably represents an unsubstituted C₅ to C₁₂ alkyl group, an unsubstituted C₅ to C₁₂ alkenyl group, an unsubstituted C₅ to C₁₂ alkynyl group or an unsubstituted C₅ to C₁₂ alkoxyl group.

The acylsulfonamide derivatives of the above general formula (I) can be produced by, for example, a method shown by the following chemical scheme: 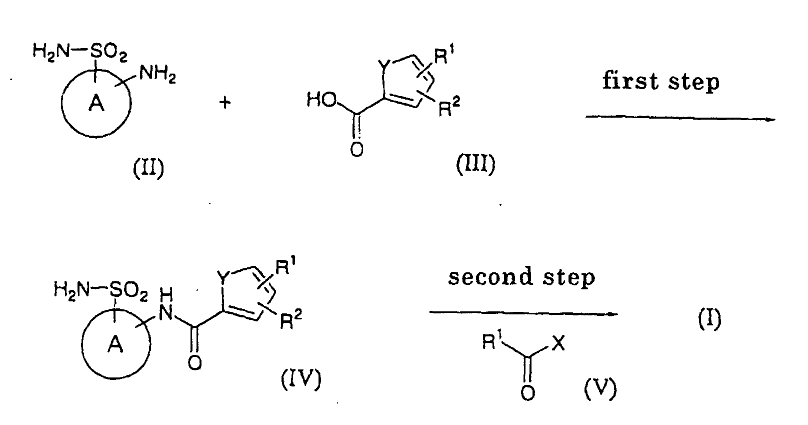 wherein R¹, R², R³, Y and ring A are as defined above, and X represents halogen atom, such as chlorine or bromine atom, succinimido group or imidazolyl group.

### (The first step)

In this step, an aminosulfonamide compound of formula (II) is condensed with a carboxylic acid of formula (III) to form a sulfonamide compound of formula (IV).

The reaction in this step can be carried out by a process wherein a condensing agent such as carbonyldiimidazole, dicyclohexylcarbodiimide or 1-ethyl-3-(3-dimethylaminopropyl)carbodiimide hydrochloride is used; a process wherein a carboxylic acid of formula (III) is converted into a corresponding acid halide with a halogenating agent such as thionyl chloride or phosphorus pentachloride and condensing it in the presence of an appropriate base; or a process wherein a carboxylic acid of formula (III) is converted into an acid anhydride with p-toluenesulfonyl chloride, ethyl chlorocarbonate, pivaloyl chloride or the like and then the acid anhydride is condensed in the presence of an appropriate base.

In carrying out the reaction, it is desirable that the aminosulfonamide compound of formula (II) and the carboxylic acid of formula (III) are used in almost equimolar amounts. Although the reaction temperature and the reaction time are not generally limited because they vary depending on the kind of the compounds, the intended compound can be obtained in a high yield by carrying out the reaction at a temperature ranging from about 0°C to around the boiling point of the solvent used for about 0.1 to 25 hours. The amount of the condensing agent is preferably about 1.2 equivalents per equivalent of the carboxylic acid of formula (III) to be reacted.

The bases usable herein include alkali metal hydrides such as sodium hydride and potassium hydride; alkali metal hydroxides such as sodium hydroxide and potassium hydroxide; alkali metal carbonates such as sodium carbonate and potassium carbonate; alkali metal hydrogencarbonates such as sodium hydrogencarbonate and potassium hydrogencarbonate; alkali metal alkoxides such as sodium methoxide, sodium ethoxide, potassium methoxide and potassium tert-butoxide; trialkylamines such as trimethylamine and triethylamine; and organic bases and inorganic bases such as pyridine, dimethylaminopyridine, picoline and lutidine. The base is used in an amount of 1 to 10 equivalents per equivalent of the carboxylic acid compound.

In this step, the reaction can be carried out in an inert solvent. The solvents include ethers such as diethyl ether, tetrahydrofuran (THF) and dioxane; aromatic hydrocarbons such as benzene, toluene and xylene; hydrocarbons such as cyclopentane and cyclohexane; halogenated hydrocarbons such as dichloromethane, dichloroethane, trichloroethane and chloroform; nitriles such as acetonitrile and propionitrile; esters such as ethyl acetate; N,N-dimethylformamide and dimethyl sulfoxide; and mixtures of them with water.

### (The second step)

In this step, a sulfonamide compound of formula (IV) is reacted with an acyl compound of formula (V) in the presence of a base to produce an acylsulfonamide compound of formula (I).

In formula (V) for the acyl compounds used in this step, X represents a halogen atom such as chlorine or bromine atom, succinimido group or imidazolyl group.

The bases usable in step 2 are the same as those usable in step 1. The amount of the base is preferably 1 to 10 equivalents per equivalent of the carboxylic acid compound.

For the reaction, the sulfonamide compound of formula (IV) and the acyl compound of formula (V) are preferably used in nearly equimolar amounts. Although the reaction temperature and the reaction time are not generally limited because they vary depending on the kind of the compounds, the intended compound can be obtained in a high yield by carrying out the reaction at a temperature ranging from about 0°C to around the boiling point of the solvent for about 0.1 to 25 hours.

The reaction can be carried out in an inert solvent. The inert solvents may be the same as those in step 1.

The intended acylsulfonamide derivatives of the above general formula (I) can be obtained by suitably combining the above-described reactions. If necessary, the reaction solution can be purified by an ordinary purification means such as filtration, decantation, extraction, washing, distillation of the solvent, column chromatography, thin-layer chromatography, recrystallization or distillation.

### Examples

The following Reference Examples and Examples will further illustrate the present invention, which by no means limit the invention. Reference Example 1: 3-Benzyloxy-N-(2-sulfamoylphenyl)benzamide:

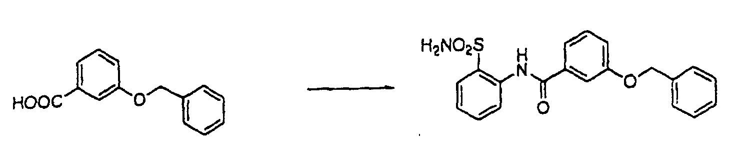

A solution of 2 g (8.8 mmol) of 3-benzyloxybenzoic acid and 8 ml of thionyl chloride in benzene (40 ml) was heated under reflux for 2 hours and then the solvent was evaporated. A solution of the residue in dioxane (10 ml) was added dropwise to a solution of 1.5 g (8.8 mmol) of 2-aminobenzenesulfonamide and 1.62 g (19 mmol) of sodium hydrogencarbonate in water - dioxane (1:1) (20 ml), and the obtained mixture was stirred at room temperature for 1 hour. After the completion of the reaction, dioxane was evaporated. 1 N HCl was added to the residue under cooling with ice to make the residue acidic. After the extraction with ethyl acetate, the extract was washed with water and saturated aqueous sodium chloride solution and then dried over anhydrous sodium sulfate. The solvent was evaporated under reduced pressure. The residue was recrystallized from acetonitrile to obtain 2.0 g (yield: 60 %) of the title compound.
¹H-NMR (CDCl₃) δ:4.86 (2H, s), 5.15 (2H, s), 7.18 (1H, dd, J = 8Hz, 1Hz), 7.26 (1H, dd d, J=8Hz, 8Hz, 2Hz), 7.25-7.48 (6H, m), 7.59 (1H, dd, J=1Hz, 1Hz), 7.63 (1H, dd, J=8 Hz, 8Hz), 7.97 (1H, dd, J = 8Hz, 2Hz), 8.56 (1 H, d, J=8Hz), 10.03 (1H, s)
IR (ν, cm⁻¹, KBr):1662, 1332, 1151
EI-MS (m/z, %) : 382 (M+, 18), 302 (6), 211 (15), 91 (100)
m. p.:181-182°C

### Reference Example 2 N-[2-(3-Benzyloxybenzamido)benzenesulfonyl]-3-benzyloxybenzamide

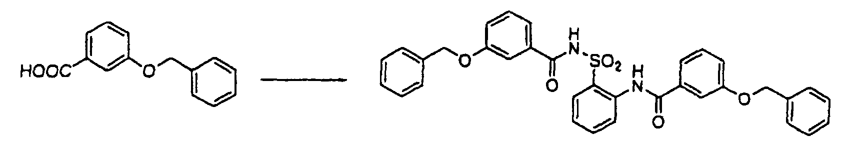

A solution of 600 mg (2.3 mmol) of 3-benzyloxybenzoic acid and 1.5 ml of thionyl chloride in benzene (10 ml) was heated under reflux for 2 hours and then the solvent was evaporated. A solution of the residue in dioxane (10 ml) was added dropwise to a solution of 248 mg (1.44 mmol) of 2-aminobenzenesulfonamide and 726 mg (5.2 mmol) of potassium carbonate in water - dioxane (1:1) (10 ml), and the obtained mixture was stirred at room temperature for 3 hour. After the completion of the reaction, dioxane was evaporated. 1 N HCl was added to the residue under cooling with ice to make the residue acidic. After the extraction with ethyl acetate, the extract was washed with water and saturated aqueous sodium chloride solution and then dried over anhydrous sodium sulfate. The solvent was evaporated under reduced pressure. The residue was recrystallized from acetonitrile to obtain 200 mg (yield: 90 %) of the title compound.
¹H-NMR (CDCl₃) δ:5.01 (2H, s), 5.13 (2H, s), 7.13-7.50 (17H, m), 7.63-7.78 (3H, m), 8.02 (1H, dd, J=8Hz, 2Hz), 8.78 (1H, d, J= 8Hz), 8.87 (1H, br-s), 10.59 (1H, s)
IR (ν, cm⁻¹, KBr):1693, 1585, 1340, 1278, 1159, 1029
EI-MS (m/z, %) : 592 (M+, 24), 368 (7), 300 (11), 212 (7), 211 (41), 181 (10), 121 (10)
m. p.:172-173°C

### Reference Example 3 N-[2-(3-Benzyloxybenzamido)benzenesulfonyl]-benzamide

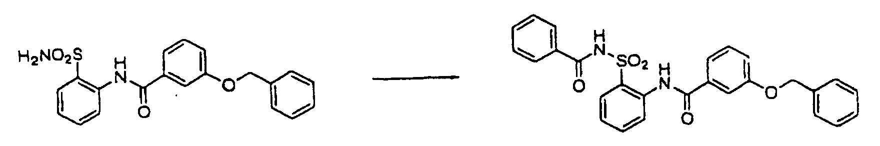

88 mg (0.78 mmol) of potassium t-butoxide was added to a solution of 300 mg (0.78 mmol) of 2-(3-benzyloxybenzamido)benzenesulfonamide produced in Reference Example 1 in anhydrous tetrahydrofuran (10 ml) under cooling with ice in nitrogen stream, and they were stirred for 1 hour. Then 111 mg (0.78 mmol) of benzoyl chloride was added to the solution, and they were stirred at room temperature for 3 hours. After the completion of the reaction, an aqueous ammonium chloride solution was added thereto to make the solution neutral. The solvent was evaporated and the residue was subjected to the extraction with ethyl acetate. The extract was washed with water and saturated aqueous sodium chloride solution and then dried over anhydrous sodium sulfate. The solvent was evaporated under reduced pressure. The residue was purified by the silica gel column chromatography to obtain 247 mg (yield: 65 %) of the title compound.
¹H-NMR (CDCl₃) δ:5.15 (2H, s), 7.17-7.4 6 (10H, m), 7.59 (1H, dd, J=8Hz, 8Hz), 7.66 -7.76 (5H, m), 8.03 (1H, d, J=8Hz), 8.78 (1 H, d, J=8Hz), 8.81 (1H, s), 10.61 (1H, s)
IR (ν, cm⁻¹, KBr):3396, 3255, 1687, 1675, 1587, 1529, 1440, 1340, 1303, 1162, 707
EI-MS (m/z, %):486 (M+, 10), 365 (6), 303 (8), 302 (5), 212 (4), 211 (16)
m. p.:201-202°C

### Reference Example 4: N-[2-(3-Benzyloxybenzamido) benzenesulfonyl] acetamide:

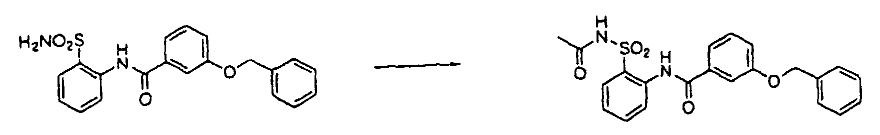

162 mg (1.44 mmol) of potassium t-butoxide was added to a solution of 400 mg (1.04 mmol) of 2-(3-benzyloxybenzamido)benzenesulfonamide prepared in Reference Example 1 in anhydrous tetrahydrofuran (10 ml) under cooling with ice in nitrogen stream, and they were stirred for 1 hour. Then 121 mg (1.54 mmol) of acetyl chloride was added to the solution, and they were stirred at room temperature for 3 hours. After the completion of the reaction, the reaction mixture was neutralized with an aqueous ammonium chloride solution. The solvent was evaporated and the residue was subjected to the extraction with ethyl acetate. The extract was washed with water and saturated aqueous sodium chloride solution and then dried over anhydrous sodium sulfate. The solvent was evaporated under reduced pressure. The residue was purified by the silica gel column chromatography to obtain 160 mg (yield: 35 %) of the title compound.
¹H-NMR (DMSO-d₆) δ:1.99 (3H, s), 5.20 (2 H, s), 7.28-7.62 (10H, m), 7.76 (1H, dd, J= 8Hz , 8Hz), 7.95 (1H, dd , J=8Hz, 1Hz), 8.41 (1H, dd, J=8Hz, 1Hz), 10.45 (1H, s), 12.58 (1H, s)
IR (ν, cm⁻¹, KBr):3342, 3118, 2871, 1708, 1660, 1585, 1531, 1444, 1162, 1029, 862, 752, 694
EI -MS (m/z , %) : 424 (M⁺, 69), 365 (13), 30 3 (27), 302 (22), 301 (10), 212 (16), 211 (71), 210 (20), 183 (16)
m. p. : 195-196°C

### Reference Example 5: N-[2-(3-Benzyloxybenzamido)benzenesulfonyl]-4-trifluoromethylbenzamide:

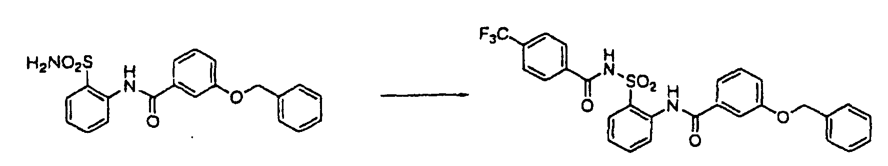

A solution of 275 mg (0.72 mmol) of 2-(3-benzyloxybenzamido)benzenesulfonamide prepared in Reference Example 1, 0.24 ml (1.44 mmol) of 4-trifluoromethylbenzyl chloride and 300 mg (2.16 mmol) of potassium carbonate in water - dioxane 1:1 (10 ml) was stirred for 18 hours. The solvent was evaporated and the residue was subjected to the extraction with ethyl acetate. The extract was washed with water and saturated aqueous sodium chloride solution and then dried over anhydrous sodium sulfate. The solvent was evaporated under reduced pressure. The residue was purified by the silica gel column chromatography to obtain 322 mg (yield: 88 %) of the title compound.
¹H-NMR(CDCl₃) δ:5.13 (2H, s), 7.18 (1H, dd, J=6Hz, 1Hz), 7.23-7.43 (7H, m), 7.64-7.72 (5H, m), 7.85 (2H, d, J=8Hz). 8.02 (1H, dd, J=6Hz, 1Hz), 8.75 (1H, dd , J=6Hz, 1Hz), 9.25 (1H, br-s), 10. 53 (1H, br-s)
IR (ν, cm⁻¹, KBr) : 1698, 1658, 1536, 1478, 1442, 1358, 1324, 1310, 1174
EI-MS (m/z, %) : 554 (M+, 14), 368 (7), 211 (15), 173 (13)
m. p. : 220-221°C

### Reference Example 6: N-[2-(3-benzyloxybenzamido)benzenesulfonyl]-4-nitrob enzamide

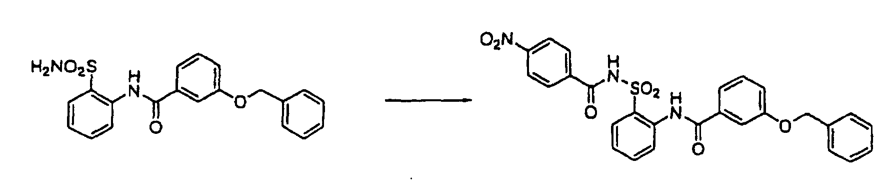

A solution of 300 mg (0.78 mmol) of 2-(3-benzyloxybenzamido)benzenesulfonamide obtained in Reference Example 1, 290 mg (1.56 mmol) of 4-nitrobenzoyl chloride and 325 mg (2.34 mmol) of potassium carbonate in water - dioxane 1:1 (10 ml) was stirred for 18 hours. The solvent was evaporated and the residue was subjected to the extraction with ethyl acetate. The extract was washed with water and saturated aqueous sodium chloride solution and then dried over anhydrous sodium sulfate. The solvent was evaporated under reduced pressure. The residue was purified by the silica gel column chromatography to obtain 320 mg (yield: 77 %) of the title compound.
¹H-NMR (CDCl₃) δ:5.14 (2H, s), 7.20 (1H, d d, J=6Hz, 1Hz), 7.22-7.46 (7H, m), 7.64 (1 H, d, J=6Hz), 7.65-7.70 (2H, m), 7.90 (2H, d, J=8Hz), 8.04 (1H, d, J=6Hz), 8.25 (2H, d, J=8Hz), 8.74 (1H, d, J = 6 Hz), 9.25 (1H, br-s), 10.53 (1H, br-s)
IR (ν, cm⁻¹, KBr) : 1696, 1662, 1608, 1474, 1442, 1344, 1320, 1276, 1250
EI-MS (m/z, %): 531 (M+, 3), 303 (3), 212 (4), 211 (12)
m. p.: 233-234°C

### Reference Example 7: N-[2-(3-benzyloxybenzamido)benzenesulfonyl]-4-methoxybenzamide

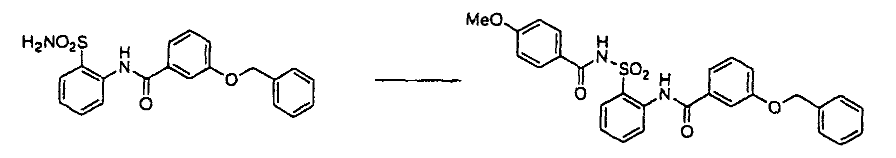

A solution of 300 mg (0.78 mmol) of 2-(3-benzyloxybenzamido)benzenesulfonamide obtained in Reference Example 1, 267 mg (1.56 mmol) of 4-methoxybenzoyl chloride and 325 mg (2.34 mmol) of potassium carbonate in water - dioxane 1:1 (10 ml) was stirred for 18 hours. The solvent was evaporated and the residue was subjected to the extraction with ethyl acetate. The extract was washed with water and saturated aqueous sodium chloride solution and then dried over anhydrous sodium sulfate. The solvent was evaporated under reduced pressure. The residue was purified by the silica gel column chromatography to obtain 282 mg (yield: 70 %) of the title compound.
¹H-NMR (CDCl₃) δ : 3.38 (3H, s), 5.14 (2H, s ), 6.90 (2H, d, J=8Hz), 7.19 (1H, dd, J=7Hz, 1Hz), 7.22-7.46 (8H, m), 7.64-7.74 (3H, m), 7.77 (1H, m), 8.02 (1H, dd, J=7Hz, 1Hz), 8.73 (1H, br-s), 8.76 (1H, dd, J=7Hz, 1Hz), 10.63 (1H, br-s)
IR (ν, cm⁻¹, KBr):1702, 1688, 1606, 1582, 1536, 1516, 1490, 1472, 1444, 1414, 1344, 1310, 1270, 1248
EI-MS (m/z, %): 516 (M+, 4), 424 (4), 303 (6), 302 (4), 212 (5), 211 (14)
m. p.:189-190°C

### Reference Example 8: N-[2-(3-Benzyloxybenzamido)benzenesulfonyl] cyclohexanamide:

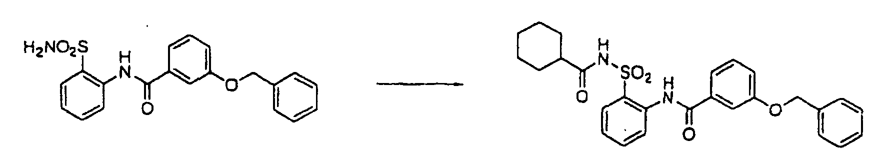

196 mg (1.56 mmol) of potassium tert-butoxide was added to a solution of 300 mg (0.78 mmol) of 2-(3-benzyloxybenzamido)benzenesulfonamide prepared in Reference Example 1 in anhydrous tetrahydrofuran (10 ml) under cooling with ice in nitrogen stream, and they were stirred for 1 hour. Then 176 mg (1.19 mmol) of cyclohexanecarbonyl chloride was added to the solution, and they were stirred at room temperature for 3 hours. After the completion of the reaction, the reaction mixture was neutralized with an aqueous ammonium chloride solution. The solution was evaporated and the residue was subjected to the extraction with ethyl acetate. The extract was washed with water and saturated aqueous sodium chloride solution and then dried over anhydrous sodium sulfate. The solvent was evaporated under reduced pressure. The residue was purified by the silica gel column chromatography to obtain 257 mg (yield: 67 %) of the title compound.
¹H-NMR (CDCl₃) δ:1.03-1.39 (4H, m), 1.5 3-1.80 (6H, m), 2.04-2.18 (1H, m), 5.13 (2 H, s), 7.17 (1H, dd , J=6Hz, 1Hz), 7.21-7.2 5 (1H, m), 7.31-7.4 3 (6H, m), 7.61-7.69 (2 H, m), 7.70-7.72 (1H, m), 7.96 (1H, dd, J = 6 Hz, 1Hz), 8.49 (1H, s), 8, 70 (1H, d, J=6Hz), 10.46 (1H, s)
IR (ν, cm⁻¹, KBr) : 1710, 1662, 1582, 1530, 1488, 1476, 1448, 1382, 1342, 1304, 1274, 1246
EI-MS (m/z, %) : 492 (M+, 82), 382 (39), 302 (45), 211 (84), 91 (100)

### Reference Example 9: N-[2-(3-Benzyloxybenzamido)benzenesulfonyl]-n-hexanamide:

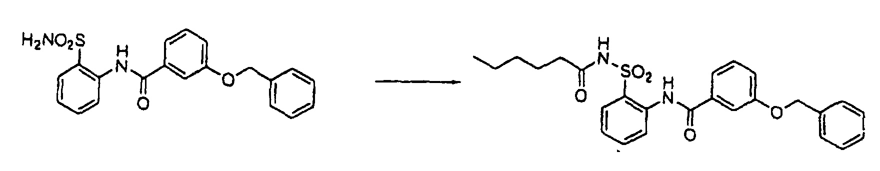

210 mg (1.67 mmol) of potassium tert-butoxide was added to a solution of 300 mg (0.78 mmol) of 2-(3-benzyloxybenzamido)benzenesulfonamide prepared in Reference Example 1 in anhydrous tetrahydrofuran (10 ml) under cooling with ice in nitrogen stream, and they were stirred for 1 hour. Then 176 mg (1.19 mmol) of n-hexanoyl chloride was added to the solution, and they were stirred at room temperature for 3 hours. After the completion of the reaction, the reaction mixture was neutralized with an aqueous ammonium chloride solution. The solution was evaporated and the residue was subjected to the extraction with ethyl acetate. The extract was washed with water and saturated aqueous sodium chloride solution and then dried over anhydrous sodium sulfate. The solvent was evaporated under reduced pressure. The residue was purified by the silica gel column chromatography to obtain 300 mg (yield: 86 %) of the title compound.
¹H-NMR (CDCl₃) δ:0.80 (3H, t, J=7Hz), 1. 13-1.23 (4H, m), 1.47-1.58 (2H, m), 2.20 (2 H, t, J=7Hz), 5.13 (2H, s), 7.16 (1H, dd, J= 6Hz, 1Hz), 7.21-7.25 (1H, m), 7.30-7.72 (6 H, m), 7.61-7.72 (3H, m), 7.96 (1H, dd, J=6 Hz, 1Hz), 8.47 (1H, br-s), 8,70 (1H, dd, J= 6Hz, 1Hz), 10.45 (1H, s)
IR (ν, cm⁻¹, KBr):1710, 1660, 1606, 1588, 1538, 1472, 1430, 1316, 1272
EI-MS (m/z, %) : 480 (M+, 57), 382 (27), 30 2 (31), 211 (71), 91 (100)
m. p. : 133-134°C

### Reference Example 10: N-[2-(3-Benzyloxybenzamido)benzenesulfonyl]-decanamide:

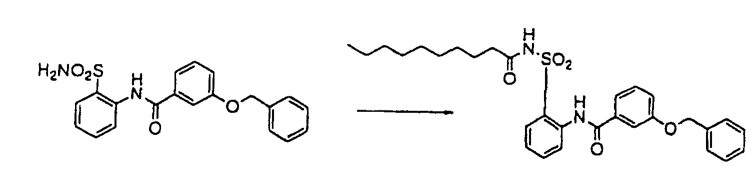

117 mg (1.04 mmol) of potassium tert-butoxide was added to a solution of 200 mg (0.52 mmol) of 3-benzyloxy-N-(2-sulfamoylphenyl)benzamide prepared in Reference Example 1 in anhydrous tetrahydrofuran (10 ml) in nitrogen stream at 0°C, and they were stirred for 1 hour. Then 0.16 ml (0.78 mmol) of decanoyl chloride was added to the solution, and they were stirred at room temperature for 3 hours. After the completion of the reaction, the reaction mixture was neutralized with an aqueous ammonium chloride solution. The solvent was evaporated under reduced pressure and the residue was subjected to the extraction with ethyl acetate. The extract was washed with water and then with saturated aqueous sodium chloride solution and then dried over anhydrous sodium sulfate. The solvent was evaporated under reduced pressure. The residue was purified by the silica gel column chromatography to obtain 150 mg (yield: 50.0 %) of the title compound.
¹H-NMR(CDCl₃) δ:0.80 (3H, t, J=7Hz), 1.1 7-1.26 (12H, m), 1.50-1.59 (2H, m), 2.21 (2 H, t, J=7Hz), 5.14 (2H, s), 7.17 (1H, dd , J = 8Hz, 1Hz), 7.24 (1H, ddd, J=8Hz, 8Hz, 1Hz), 7.31-7.48 (8H, m), 7.61-7.72 (3H, m), 7.9 8 (1H, dd, J=8Hz, 2Hz), 8.30 (1H,br-s), 8.72 (1H, d, J=8Hz), 10.43 (1H, s)
IR (ν, cm⁻¹, KBr):1704, 1662, 1606, 1588, 1540, 1472, 1342, 1166
FAB-MS (neg:m/z, %):535 ([M-H] +100)
m. p.:85-86°C

### Reference Example 11: N-[2-(3-Benzyloxybenzamido)benzenesulfonyl] pentafluorobenzamide:

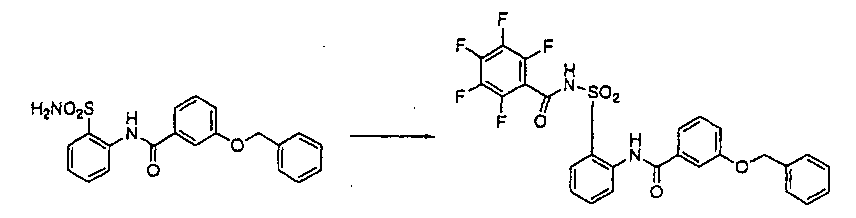

300 mg (0.78 mmol) of 3-benzyloxy-N-(2-sulfamoylphenyl)benzamide obtained in Reference Example 1, 271 mg (1.18 mmol) of pentafluorobenzoyl chloride and 374 mg (2.70 mmol) of potassium carbonate were dissolved in a mixed solvent (10 ml) of water - dioxane 1:1.The obtained solution was stirred at room temperature for 18 hours. After the completion of the reaction, the solvent was evaporated under reduced pressure and the residue was subjected to the extraction with ethyl acetate. The extract was washed with water and then with saturated aqueous sodium chloride solution and then dried over anhydrous sodium sulfate. The solvent was evaporated under reduced pressure. The residue was purified by the silica gel column chromatography to obtain 350 mg (yield: 77.0 %) of the title compound.
¹H-NMR (CDCl₃) δ:5.14 (2H, s), 7.19 (1H, ddd, J=8Hz, 2Hz, 1Hz), 7.31-7.48 (7H, m), 7.60 (1H, ddd, J=8Hz, 2Hz, 1Hz), 7.66 (1H, dd, J=2Hz, 1Hz), 7.73 (1H, ddd, J=8Hz, 8Hz,
1Hz), 8.06 (1H, dd, J=8Hz, 1Hz), 8.76 (1H, dd, J=8Hz, 1Hz), 10.31 (1H, s)
EI-MS (m/z, %):576 (m+, 20), 211 (14), 91 (100)
IR (ν, cm⁻¹, KBr):1704, 1654, 1582, 1536, 1506, 1440

### Reference Example 12: N-[2-(3-Benzyloxybenzamido)benzenesulfonyl]-2,4-difluorobenzamide:

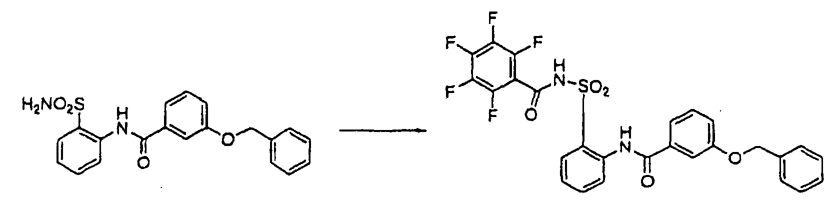

300 mg (0.78 mmol) of benzyloxy-N-(2-sulfamoylphenyl)benzamide, 273 mg (1.55 mmol) of 2,4-difluorobenzoyl chloride and 374 mg (2.70 mmol) of potassium carbonate were dissolved in a mixed solvent (10 ml) of water - dioxane (1:1). The obtained solution was stirred at room temperature for 18 hours. After the completion of the reaction, the solvent was evaporated under reduced pressure. The residue was dissolved in ethyl acetate. The resulting solution was washed with water and then with saturated aqueous sodium chloride solution and then dried over anhydrous sodium sulfate. The solvent was evaporated under reduced pressure. The residue was purified by the silica gel column chromatography to obtain 350 mg (yield: 86.0 %) of the title compound.
¹H-NMR (CDCl₃) δ:5.15 (2H, s), 6.92 (1H, ddd, J=8Hz, 8Hz, 2Hz), 7.05 (1H, ddd, J=8H z, 8Hz, 2Hz), 7.17 (1H, ddd, J=8Hz, 8Hz, 1 Hz), 7.22 (1H, ddd, J=8Hz, 2Hz, 1Hz), 7.30 -7.50 (7H, m), 7.58 (1H, dd, J=8Hz, 1Hz), 7. 70 (1H, dd, J=2Hz, 1Hz) 7.73-7.78 (1H, m), 7.83 (1H, dd, J=8Hz, 2Hz), 8.41 (1H, dd, J =8Hz, 1Hz), 11.36 (1H, s)
IR (ν, cm⁻¹, KBr):1678, 1608, 1580, 1546, 1498
FAB-MS (neg:m/z, %):521 ([M-H]+100)
m. p.:208-209°C

### Reference Example 13: 3-(4-t-Butylbenzyloxy-N-(2-sulfamoylphenyl) benzamide:

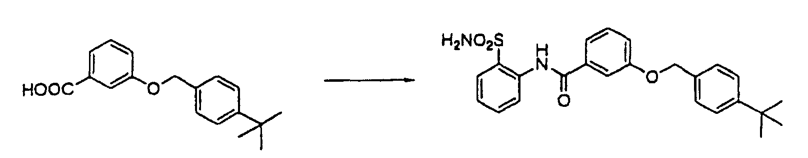

A solution of 4.00 g (14.0 mmol) of 3-(4-t-butylbenzyloxy)benzoic acid and 3 ml of thionyl chloride in benzene (30 ml) was heated under reflux for 2 hours and then the solvent was evaporated under reduced pressure. The residue was dissolved in methylene chloride (30 ml); and the obtained solution was added dropwise to a solution of 2.42 g (14.0 mmol) of 2-aminobenzenesulfonamide in pyridine (50 ml) under cooling with ice. After stirring at room temperature for 18 hours, methylene chloride was evaporated under reduced pressure. The residue was dissolved in ethyl acetate and then the obtained solution was washed with 1 N aqueous hydrochloric acid solution, water and saturated aqueous sodium chloride solution in that order. After drying over anhydrous sodium sulfate, the solvent was evaporated under reduced pressure. The obtained crude crystals were recrystallized from a solvent mixture of ether - hexane to obtain 4.2 g (yield: 68.0 %) of the title compound.
¹H-NMR (CDCl₃) δ : 1.38 (9H, s), 4.92 (2H, s) , 5.09 (2H, s), 7.16 (1H, ddd, J=8Hz, 2Hz, 1Hz), 7.23 (1H, ddd, J=8Hz, 8Hz, 2Hz), 7.3 6-7.44 (5H, m), 7.49 (1H, dd, J=8Hz, 1Hz), 7.56-7.63 (2H, m), 7.94 (1H, dd , J=8Hz, 2 Hz), 8.53 (1H, dd, J=8Hz, 1Hz), 10.04 (1H, s)
EI-MS (m/z, %) : 438 (m+, 37), 147 (100), 132 (84), 117 (65)
IR (ν, cm⁻¹, KBr):1674, 1658, 1586, 1538, 1446, 1332, 1168
m. p.:117-118°C

### Reference Example 14: N-[2-[3-(4-t-butylbenzyloxy)benzamido] benzenesulfonyl]pivalamide:

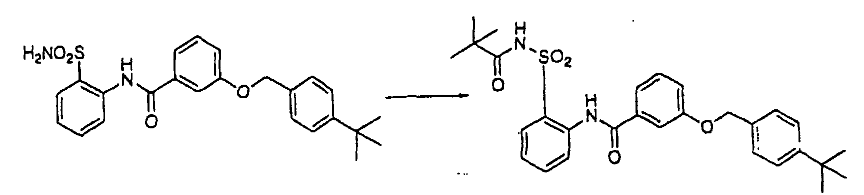

0.09 ml (0.75 mmol) of pivaloyl chloride was added to a solution of 300 mg (0.68 mmol) of 3-(4-t-butylbenzyloxy)-N-(2-sulfamoylphenyl)benzamide prepared in Reference Example 13 and 167 mg (1.36 mmol) of 4-dimethylaminopyridine in anhydrous tetrahydrofuran (10 ml) at 0°C in nitrogen stream. The obtained mixture was stirred at room temperature for 1 hour and then the solvent was evaporated under reduced pressure. The residue was dissolved in ethyl acetate and then the obtained solution was washed with water, an aqueous potassium hydrogensulfate solution and saturated aqueous sodium chloride solution in that order. After drying over anhydrous sodium sulfate, the solvent was evaporated under reduced pressure. The residue was purified by the silica gel chromatography to obtain 284 mg (yield: 80.0 %) of the title compound.
¹H-NMR (CDCl₃) δ:1.14 (9H, s), 1.33 (9H, s) , 5.10 (2H, s), 7.18 (1H, dd, J=8Hz, 2Hz), 7. 25 (1H, ddd , J=8Hz, 8Hz, 2Hz), 7,38-7.45 (10H, m), 7.63 (1H, dd, J=8Hz, 2Hz), 7.67 (1H, ddd , J=8Hz, 8Hz, 2Hz), 7.72-7.75 (1H, m), 7.97 (1H, dd, J=8Hz, 2Hz), 8.27 (1H, br -s), 8.72 (1H, dd, J=8Hz, 2Hz), 10,41 (1H, s)
IR (ν, cm⁻¹, KBr):1702, 1660, 1580, 1538, 1478, 1448, 1342, 1312, 1292
EI-MS (m/z, %) : 522 (m+, 72), 147 (100)
m. p.:193-194°C

### Reference Example 15: N-[2-[3-(4-t-butylbenzyloxy)benzamido] benzenesulfonyl]cinnamamide:

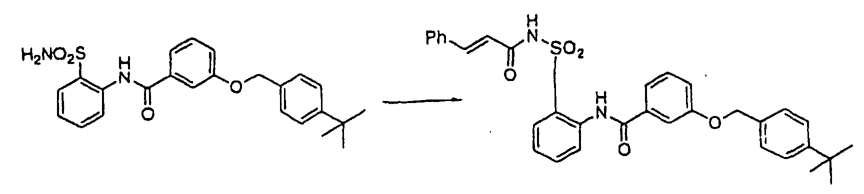

126 mg (0.75 mmol) of cinnamoyl chloride was added to a solution of 300 mg (0.68 mmol) of 3-(4-t-butylbenzyloxy)-N-(2-sulfamoylphenyl)benzamide prepared in Reference Example 13 and 167 mg (1.36 mmol) of 4-dimethylaminopyridine in anhydrous tetrahydrofuran (10 ml) at 0°C in nitrogen stream. The obtained mixture was stirred at room temperature for 1 hour and then the solvent was evaporated under reduced pressure. The residue was dissolved in ethyl acetate and then the obtained solution was washed with water, an aqueous potassium hydrogensulfate solution and saturated aqueous sodium chloride solution in that order. After drying over anhydrous sodium sulfate, the solvent was evaporated under reduced pressure. The obtained crude crystals were washed with methanol to obtain 370 mg (yield: 95.0 %) of the title compound.
¹H-NMR (CDCl₃) δ:1.31 (9H, s), 5.11 (2H, s) , 6.31 (1H, d, J=16Hz), 7.17 (1H, ddd, J=8 Hz, 2Hz, 1Hz), 7.24 (1H, ddd, J=8Hz, 8Hz, 1Hz), 7,31-7.44 (10H, m), 7.64-7.70 (3H, m), 7.77-7.78 (1H, m), 8.01 (1H, dd, J=8Hz, 2Hz), 8.74 (1H, dd , J=8Hz, 1Hz), 10,60 (1H, s)
IR (ν, cm⁻¹, KBr):1688, 1668, 1630, 1582, 1534, 1476, 1442, 1334, 1310, 1272
EI-MS (m/z, %):568 (m+, 3), 437 (3), 147 (100)
m. p.:110-111°C

### Reference Example 16: N-[2-(3-benzyloxybenzamido]benzenesulfonyl] oleinamide:

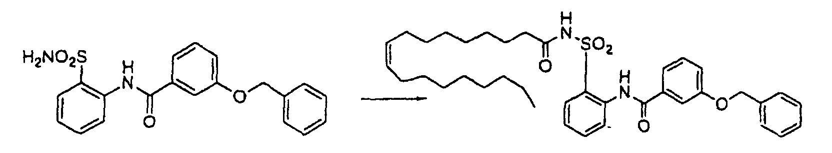

787 mg (2.62 mmol) of oleyl chloride was added to a solution of 500 mg (1.30 mmol) of 3-benzyloxy-(2-sulfamoylphenyl)benzamide prepared in Reference Example 1 and 480 mg (3.93 mmol) of 4-dimethylaminopyridine in anhydrous tetrahydrofuran (10 ml) at 0°C in nitrogen atmosphere. The obtained mixture was stirred at room temperature for 1 hour and then the solvent was evaporated under reduced pressure. The residue was dissolved in ethyl acetate and then the obtained solution was washed with water, an aqueous potassium hydrogensulfate solution and saturated aqueous sodium chloride solution in that order. After drying over anhydrous sodium sulfate, the solvent was evaporated under reduced pressure. The residue was purified by the silica gel chromatography to obtain 625 mg (yield: 74.0 %) of the title compound.
¹H-NMR(CDCl₃) δ : 0.87 (3H, t, J=7Hz), 1.1 5-1.38 (19H, m), 1.45-1.62 (4H, m), 1.85-2.01 (3H, m), 2.20 (2H, t, J=7Hz), 5.14 (2H, s), 5.30-5.42 (2H, m), 7.17 (1H, ddd, J=8H z, 2Hz, 1Hz), 7.50 (1H, ddd, J=8Hz, 8Hz, 1Hz), 7.31-7.48 (6H, m), 7.61-7.72 (3H, m), 7.78 (1H, dd, J=8Hz, 2Hz), 8.31 (1H, br-s), 8.72 (1H, dd, J=8Hz, 1Hz), 10.43 (1H, s)
IR (ν, cm⁻¹, KBr):1708, 1660, 1606, 1588, 1542, 1472, 1448, 1338
EI-MS (m/z, %):646 (m+, 14), 383 (57), 302 (12), 211 (97), 91 (100)
m. p.:82-83°C

### Reference Example 17: N-[2-(3-benzyloxybenzamido]benzenesulfonyl] linolamide:

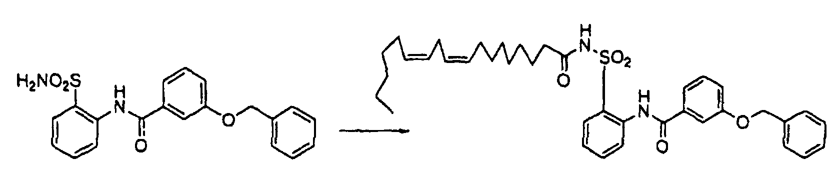

A solution of 440 mg (1.57 mmol) of linolic acid and 3 ml of thionyl chloride in benzene (30 ml) was heated under reflux for 2 hours. The solvent was evaporated under reduced pressure. The residue was dissolved in anhydrous tetrahydrofuran (10 ml). The obtained solution was added dropwise to a solution of 500 mg (1.30 mmol) of 3-benzyloxy-N-(2-sulfamoylphenyl)benzamide prepared in Reference Example 1 and 352 mg (2.88 mmol) of 4-dimethylaminopyridine in anhydrous tetrahydrofuran (10 ml) under cooling with ice. The obtained mixture was stirred at room temperature for 1 hour. The solvent was evaporated under reduced pressure. The residue was dissolved in ethyl acetate and then the obtained solution was washed with water, an aqueous potassium hydrogensulfate solution and saturated aqueous sodium chloride solution in that order. After drying over anhydrous sodium sulfate, the solvent was evaporated under reduced pressure. The residue was purified by the silica gel chromatography to obtain 625 mg (yield: 74.0 %) of the title compound.
¹H-NMR (CDCl₃) δ:0.88 (3H, t, J=7Hz), 1.1 0-1.38 (12H, m), 1.40-1.70 (4H, m), 1.93-2.10 (4H, m), 2.20 (2H, t, J=8Hz), 2.74 (2H, dd, J=6Hz, 6Hz), 5.13 (2H, s), 5.25-5.42 (4H, m), 7.16 (1H, dd, J=8Hz, 2Hz), 7.24 (1H, ddd, J=8Hz, 8Hz, 1Hz), 7.30-7.50 (6H, m), 7.60-7.71 (3H, m), 7.96 (1H, dd, J=8Hz, 2Hz), 8.54 (1H, br-s), 8.70 (1H, dd, J=8Hz, 1Hz), 10.45 (1H, s)
IR (ν, cm⁻¹, KBr):1714, 1660, 1606, 1588, 1540, 1472, 1448, 1338
FAB-MS (neg:m/z, %):643 ([M-H]+100)

### Reference Example 18: 3-Benzyloxy-4-nitro-N-(2-sulfamoylphenyl) benzamide:

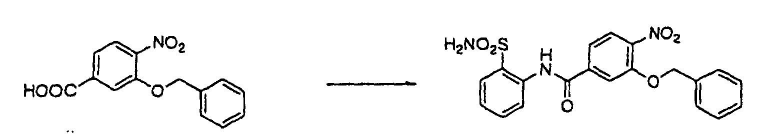

A solution of 2.00 g (7.30 mmol) of 3-benzyloxy-4-nitrobenzoic acid and 3 ml of thionyl chloride in benzene (30 ml) was heated under reflux for 2 hours. The solvent was evaporated under reduced pressure. The residue was dissolved in methylene chloride (30 ml). The obtained solution was added dropwise to a solution of 1.38 g (8.00 mmol) of 2-aminobenzenesulfonamide in pyridine (50 ml) under cooling with ice. The obtained mixture was stirred at room temperature for 18 hours. Methylene chloride was evaporated under reduced pressure. The residue was dissolved in ethyl acetate and then the obtained solution was washed with 1 N aqueous hydrochloric acid solution, water and saturated aqueous sodium chloride solution in that order. After drying over anhydrous sodium sulfate, the solvent was evaporated under reduced pressure. The obtained crude crystals were washed with methanol to obtain 2.50 g (yield: 80.0 %) of the title compound.
¹H-NMR (DMSO-d₆) δ:5.41 (2H, s), 7.34-7.52 (6H, m), 7.61 (1H, dd, J=8Hz, J=2Hz), 7.69 (1H, ddd, J=8Hz, J=8Hz, J=2Hz), 7.91-7.96 (2H, m) 8.11 (1H, d, J=8Hz), 8.3 3 (1H, d, J=8Hz), 10.41 (1H, s)

### Reference Example 19 N-[2-(3-benzyloxy-4-nitrobenzamido) benzenesulfonyl] decanamide:

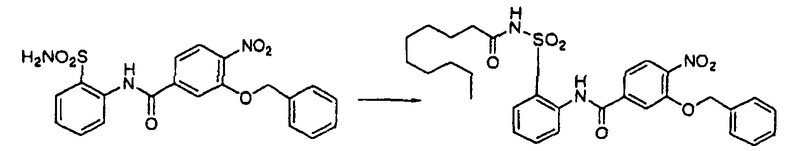

0.24 ml (1.16 mmol) of decanoyl chloride was added to a solution of 435 mg (1.02 mmol) of 3-benzyloxy-4-nitro-N-(2-sulfamoylphenyl)benzamide prepared in Reference Example 18 and 274 mg (2.23 mmol) of 4-dimethylaminopyridine in anhydrous tetrahydrofuran (10 ml) at 0°C in nitrogen stream. The obtained mixture was stirred at room temperature for 1 hour and then the solvent was evaporated under reduced pressure. The residue was dissolved in ethyl acetate and then the obtained solution was washed with water, an aqueous potassium hydrogensulfate solution and saturated aqueous sodium chloride solution in that order. After drying over anhydrous sodium sulfate, the solvent was evaporated under reduced pressure. The residue was purified by the silica gel chromatography to obtain 490 mg (yield: 83.0 %) of the title compound.
¹H-NMR (CDCl₃) δ:0.86 (3H, t, J=7Hz), 1.10-1.30 (12H, m), 1.48-1.65 (2H, m), 2.25 (2H, t, J=7Hz), 5.32 (2H, s), 7.24-7.43 (4H, m), 7.47-7.51 (2H, m), 7.68-7.74 (2H, m), 7.92-7.99 (3H, m), 8.71 (1H, dd, J=8Hz, 2Hz), 10.64 (1H, s)
IR (ν, cm⁻¹, KBr):1722, 1668, 1614, 1588, 1538, 1472, 1444, 1404, 1342, 1322, 1292 EI-MS (m/z, %) 581 (m+, 2), 321 (11), 211 (2), 91 (100)
m. p.:129-130°C

### Reference Example 20: 3-(4-Chlorobenzyloxy)-N-(2-sulfamoylphenyl) benzamide:

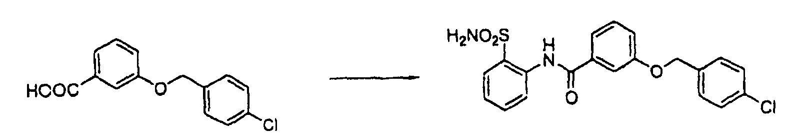

A solution of 400 g (15.2 mmol) of 3-(4-chlorobenzyloxy)benzoic acid and 3 ml of thionyl chloride in benzene (30 ml) was heated under reflux for 2 hours. The solvent was evaporated under reduced pressure. The residue was dissolved in methylene chloride (30 ml). The obtained solution was added dropwise to a solution of 2.75 g (16.0 mmol) of 2-aminobenzenesulfonamide in pyridine (50 ml) under cooling with ice. The obtained mixture was stirred at room temperature for 18 hours. Methylene chloride was evaporated under reduced pressure. The residue was dissolved in ethyl acetate and then the obtained solution was washed with 1 N aqueous hydrochloric acid solution, water and saturated aqueous sodium chloride solution in that order. After drying over anhydrous sodium sulfate, the solvent was evaporated under reduced pressure. The obtained crude crystals were washed with methanol to obtain 4.70 g (yield: 74.0 %) of the title compound.
¹H-NMR (DMSO-d₆) δ:5.20 (2H, s), 7.27-7.31 (1H, m), 7.34 (1H, ddd, J=8Hz, 8Hz, 2Hz), 7.45-7.56 (7H, m) 7.65 (1H, ddd, J=8Hz, 8Hz, 2Hz) 7.77 (2H, s), 7.92 (1H, dd, J=8Hz, 2Hz), 8.47 (1H, dd, J=8Hz, 1Hz), 10.38 (1H, s)
EI-MS (m/z, %):418 (m+2, 6) 416 (m+, 17)
IR (ν, cm⁻¹, KBr):1672, 1586, 1542, 1446, 1154
m. p.:174-175°C

### Reference Example 21: N-[2-[3-(4-Chlorobenzyloxy)benzamido] benzenesulfonyl] acetamide:

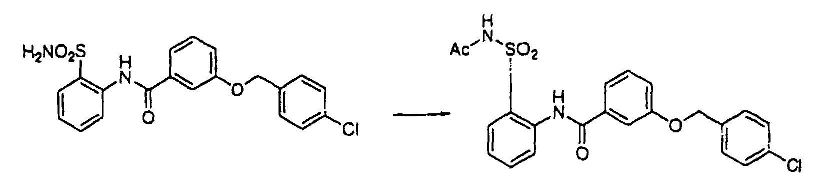

0.12 ml (1.30 mmol) of acetic acid anhydride was added to a solution of 500 mg (1.20 mmol) of 3-(4-chlorobenzyloxy)-N-(2-sulfamoylphenyl)benzamide prepared in Reference Example 20 and 293 mg (2.40 mmol) of 4-dimethylaminopyridine in anhydrous tetrahydrofuran (10 ml) at 0°C in nitrogen stream. The obtained mixture was stirred at room temperature for 1 hour and then the solvent was evaporated under reduced pressure. The residue was dissolved in ethyl acetate and then the obtained solution was washed with water, an aqueous potassium hydrogensulfate solution and saturated aqueous sodium chloride solution in that order. After drying over anhydrous sodium sulfate, the solvent was evaporated under reduced pressure. The residue was recrystallized from a solvent mixture of ethyl acetate - diethyl ether to obtain 290 mg (yield: 52.7 %) of the title compound.
¹H-NMR (CDCl₃) δ:2.06 (3H, s), 5.11 (2H. s) , 7.16 (1H, ddd, J=8Hz, J=2Hz, J=1Hz), 7.23-7.29 (1H, m), 7.34-7.44 (5H, m), 7.61-7.72 (3H, m), 7.98 (1H, dd, J=8Hz, 1Hz), 8.18 (1H, br-s), 8.73 (1H, dd , J=8Hz, 1Hz), 10.41 (1H, br-s)
IR (ν, cm⁻¹, KBr):3768, 3108, 2872, 1716, 1666, 1580, 1538, 1478, 1448.
EI-MS (m/z, %):460 (9), 458 (23), 336 (5), 245 (11), 183 (6), 127 (53), 125 (100), 92 (10) m. p.:176-179°C

### Reference Example 22: N-[2-[3-(4-Chlorobenzyloxy)benzamido] benzenesulfonyl]hexanamide:

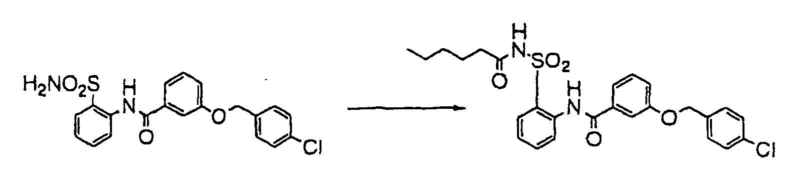

0.18 mg (1.30 mmol) of hexanoyl chloride was added to a solution of 500 mg (1.2 mmol) of 3-(4-chlorobenzyloxy)-N-(2-sulfamoylphenyl)benzamide prepared in Reference Example 20 and 293 mg (2.40 mmol) of 4-dimethylaminopyridine in anhydrous tetrahydrofuran (10 ml) at 0°C in nitrogen stream. The obtained mixture was stirred at room temperature for 1 hour and then the solvent was evaporated under reduced pressure. The residue was dissolved in ethyl acetate and then the obtained solution was washed with water, an aqueous potassium hydrogensulfate solution and saturated aqueous sodium chloride solution in that order. After drying over anhydrous sodium sulfate, the solvent was evaporated under reduced pressure. The residue was purified by the silica gel chromatography to obtain 600 mg (yield: 97 %) of the title compound.
¹H-NMR (CDCl₃)δ:0.81 (3H, t, J=7Hz), 1.1 3-1.28 (4H, m), 1.48-1.60 (2H, m), 2.22 (2H, t, J=7Hz), 5.11 (2H, s), 7.15 (1H, ddd, J =8Hz, 2Hz, 1Hz), 7.23-7.28 (1H, m), 7.34-7.43 (5H, m), 7.62-7.70 (1H, m), 7.97 (1H, dd, J=8Hz, 2Hz), 8.21 (1H, br-s), 8.73 (1H, dd, J=8Hz, 1Hz), 10.45 (1H, br-s)
IR (ν, cm⁻¹, KBr):3392, 3080, 2952, 2932, 2868, 1720, 1658, 1580, 1536, 1486, 1474, 1448, 1412.
EI-MS (m/z, %):526 (4), 514 (8), 336 (4), 24 5 (9), 183 (4), 127 (31), 125 (100)
m. p.:148-149°C

### Reference Example 23: N-[2-[3-(4-Chlorobenzyloxy)benzamido] benzenesulfonyl]decanamide:

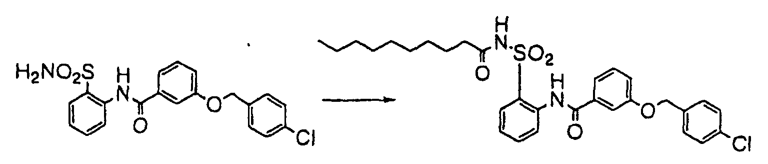

0.27 mg (1.30 mmol) of decanoyl chloride was added to a solution of 500 mg (1.20 mmol) of 3-(4-chlorobenzyloxy)-N-(2-sulfamoylphenyl)benzamide prepared in Reference Example 20 and 293 mg (2.40 mmol) of 4-dimethylaminopyridine in anhydrous tetrahydrofuran (10 ml) at 0°C in nitrogen stream. The obtained mixture was stirred at room temperature for 1 hour and then the solvent was evaporated under reduced pressure. The residue was dissolved in ethyl acetate and then the obtained solution was washed with water, an aqueous potassium hydrogensulfate solution and saturated aqueous sodium chloride solution in that order. After drying over anhydrous sodium sulfate, the solvent was evaporated under reduced pressure. The residue was purified by the silica gel chromatography to obtain 640 mg (yield: 93.07 %) of the title compound.
¹H-NMR (CDCl₃) δ:0. 86 (3H, t, J=7Hz), 1.15-1.30 (12H, m), 1.48-1.59 (2H, m), 2.22 (2H, t, J=7Hz), 5.11 (2H, s), 7.15 (1H, ddd, J =8Hz, 2Hz, 1Hz), 7.23-7.28 (1H, m), 7.34-7.44 (5H, m), 7.62-7.70 (3H, m), 7.97 (1H, dd, J=8Hz, 1Hz), 8.18 (1H, br-s), 8.73 (1H, dd , J=8Hz, 1Hz), 10.45 (1H, br-s)
IR (ν, cm⁻¹, KBr):3368, 3032, 2920, 2852, 1702, 1662, 1604, 1586, 1542, 1494, 1472, 1448, 1438.
EI-MS (m/z, %):572 (2), 570 (4), 336 (4), 24 5 (10), 183 (3), 127 (32), 125 (100)
m. p.:159-161°C

### Reference Example 24: 3-(4-Nitrobenzyloxy)-N-(2-sulfamoylphenyl) benzamide:

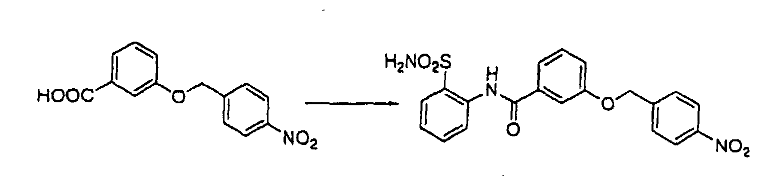

A solution of 1.40 g (5.1 mmol) of 3-(4-nitrobenzyloxy)benzoic acid and 2 ml of thionyl chloride in benzene (20 ml) was heated under reflux for 2 hours. The solvent was evaporated under reduced pressure. The residue was dissolved in methylene chloride (20 ml). The obtained solution was added dropwise to a solution of 0.97 g (5.6 mmol) of 2-aminobenzenesulfonamide in pyridine (30 ml) under cooling with ice. The obtained mixture was stirred at room temperature for 18 hours. Methylene chloride was evaporated under reduced pressure. The residue was dissolved in ethyl acetate and then the obtained solution was washed with 1 N aqueous hydrochloric acid solution, water and saturated aqueous sodium chloride solution in that order. After drying over anhydrous sodium sulfate, the solvent was evaporated under reduced pressure. The obtained crude crystals were washed with methanol to obtain 1.9 g (yield: 87.0 %) of the title compound.
¹H-NMR (DMSO-d₆) δ:5.39 (2H, s), 7.30-7.38 (2H, m), 7.50-7.58 (3H, m), 7.65 (1H, ddd, J=8Hz, 8Hz, 2Hz), 7.75-7.80 (4H, m), 7.91 (1H, dd, J=8Hz, 2Hz), 8.25-8.30 (2H, m), 8.46 (1H, dd , J=8, 1Hz), 10.38 (1H, s)
EI-MS (m/z, %): 427 (m+, 23), 347 (82), 256 (100), 121 (50)
IR (ν, c m⁻¹, KBr) : 1658, 1590, 1530, 1446, 1344, 1152, 1046
m. p. : 177-178°C

### Reference Example 25: N-[2-[3-(4-Nitrobenzyloxy)benzamido] benzenesulfonyl] acetamide:

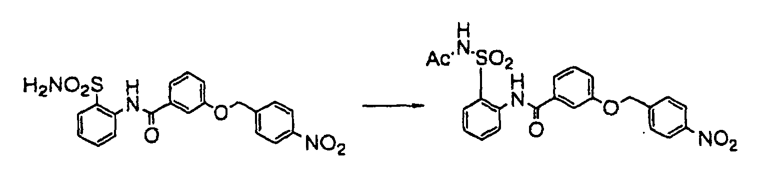

0.07 mg (0.80 mmol) of acetic acid anhydride was added to a solution of 300 mg (0.70 mmol) of 3-(4-nitrobenzyloxy)-N-(2-sulfamoylphenyl)benzamide prepared in Reference Example 24 and 171 mg (1.40 mmol) of 4-dimethylaminopyridine in anhydrous tetrahydrofuran (10 ml) at 0°C in nitrogen stream. The obtained mixture was stirred at room temperature for 1 hour and then the solvent was evaporated under reduced pressure. The residue was dissolved in ethyl acetate and then the obtained solution was washed with water, an aqueous potassium hydrogensulfate solution and saturated aqueous sodium chloride solution in that order. After drying over anhydrous sodium sulfate, the solvent was evaporated under reduced pressure. The residue was purified by the silica gel chromatography to obtain 290 mg (yield: 89.8 %) of the title compound.
¹H-NMR (CDCl₃) δ:2.05 (3H, s), 5.25 (2H, s) , 7.15-7.19 (1H, m), 7.23-7.29 (1H, m), 7.40-7.45 (1H, m), 7.60-7.71 (5H, m), 7.96 (1H, dd, J=8Hz, 1Hz), 8.22-8.27 (2H, m), 8.47 (1H, br-s), 8.72 (1H, dd, J=8Hz, 1Hz), 10.45 (1H, br-s)
IR (ν, cm⁻¹, KBr):3384, 3124, 2856, 1714, 1660, 1580, 1532, 1520, 1490, 1476, 1446 EI-MS (m/z, %) : 469 (9), 347 (15), 256 (27), 182 (8), 154 (4), 125 (100), 121 (27)
m. p. : 189-192°C

### Reference Example 26: N-[2-[3-(4-Nitrobenzyloxy)benzamido] benzenesulfonyl]hexanamide:

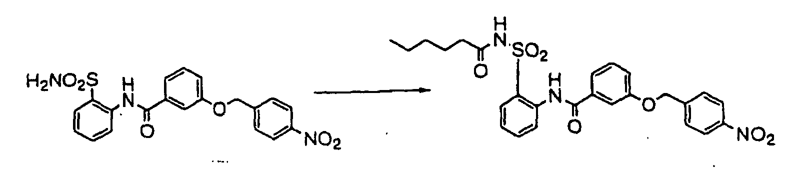

0.11 mg (0.80 mmol) of hexanoyl chloride was added to a solution of 300 mg (0.70 mmol) of 3-(4-nitrobenzyloxy)-N-(2-sulfamoylphenyl)benzamide prepared in Reference Example 24 and 171 mg (1.40 mmol) of 4-dimethylaminopyridine in anhydrous tetrahydrofuran (10 ml) at 0°C in nitrogen stream. The obtained mixture was stirred at room temperature for 1 hour and then the solvent was evaporated under reduced pressure. The residue was dissolved in ethyl acetate and then the obtained solution was washed with water, an aqueous potassium hydrogensulfate solution and saturated aqueous sodium chloride solution in that order. After drying over anhydrous sodium sulfate, the solvent was evaporated under reduced pressure. The residue was purified by the silica gel chromatography to obtain 320 mg (yield: 86.8 %) of the title compound.
¹H-NMR(CDCl₃) δ:0.81 (3H, t, J=7Hz), 1.13-1.28 (4H, m), 1.49-1.59 (2H, m), 2.23 (2H, t, J=7Hz), 5.25 (2H, s), 7.15-7.20 (1H, m), 7.23-7.2 9 (1H, m), 7.61-7.71 (5H, m), 7.97 (1H, dd, J=8Hz, 1Hz), 8.22-8.29 (3H, m), 8.73 (1H, dd, J=8Hz, 1Hz), 10.48 (1H, br-s)
IR (ν, cm⁻¹, KBr):3372, 3064, 2956, 2932, 2860, 1714, 1664, 1604, 1586, 1526, 1490, 1472, 1446.
EI-MS (m/z, %) : 525 (32), 411 (6), 347 (86), 256 (100), 182 (20), 121 (50), 92 (17)
m. p.:138-141°C

### Reference Example 27: N-[2-[3-(4-nitrobenzyloxy)benzamido] benzenesulfonyl]decanamide:

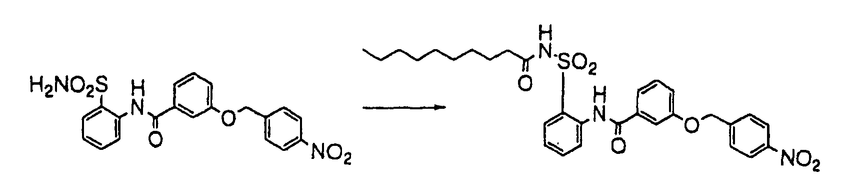

0.167 mg (0.80 mmol) of decanoyl chloride was added to a solution of 300 mg (0.70 mmol) of 3-(4-nitrobenzyloxy)-N-(2-sulfamoylphenyl)benzamide prepared in Reference Example 24 and 171 mg (1.40 mmol) of 4-dimethylaminopyridine in anhydrous tetrahydrofuran (10 ml) at 0°C in nitrogen stream. The obtained mixture was stirred at room temperature for 1 hour and then the solvent was evaporated under reduced pressure. The residue was dissolved in ethyl acetate and then the obtained solution was washed with water, an aqueous potassium hydrogensulfate solution and saturated aqueous sodium chloride solution in that order. After drying over anhydrous sodium sulfate, the solvent was evaporated under reduced pressure. The residue was purified by the silica gel chromatography to obtain 400 mg (yield: 97.9 %) of the title compound.
¹H-NMR(CDCl₃) δ:0.86 (3H, t, J=7Hz), 1.15-1.30 (12H, m), 1.48-1.59 (2H, m), 2.23 (2H, t, J=7Hz), 5.25 (2H, s), 7.15-7.20 (1H, m), 7.23-7.29 (1H, m), 7.40-7.46 (1H, m), 7.61-7.71 (5 H, m), 7.96 (1H, dd , J=8Hz, 1Hz), 8.22 (1H, br-s), 8.23-8.29 (2H, m), 8.73 (1H, dd, J=8Hz, 1Hz), 10. 48 (1H, br-s)
IR (ν, cm⁻¹, KBr) : 3376, 3068, 2924, 2852, 1704, 1668, 1606, 1588, 1256, 1490, 1472, 1446
EI-MS (m/z, %):581 (20), 411 (8), 347 (87), 256 (100), 182 (19), 136 (22)
m. p.:145-148°C

### Reference Example 28: 3-(4-Methoxybenzyloxy)-N-(2-sulfamoylphenyl) benzamide:

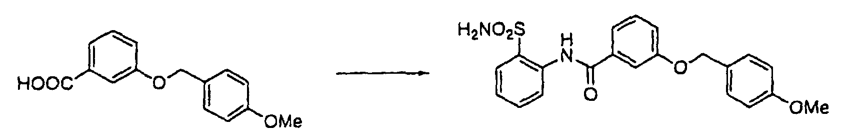

A solution of 4.00 g (15.5 mmol) of 3-(4-methoxybenzyloxy)benzoic acid and 3 ml of thionyl chloride in benzene (30 ml) was heated under reflux for 2 hours. The solvent was evaporated under reduced pressure. The residue was dissolved in methylene chloride (30 ml). The obtained solution was added dropwise to a solution of 2.93 g (17.0 mmol) of 2-aminobenzenesulfonamide in pyridine (50 ml) under cooling with ice. The obtained mixture was stirred at room temperature for 18 hours. Methylene chloride was evaporated under reduced pressure. The residue was dissolved in ethyl acetate and then the obtained solution was washed with 1 N aqueous hydrochloric acid solution, water and saturated aqueous sodium chloride solution in that order. After drying over anhydrous sodium sulfate, the solvent was evaporated under reduced pressure. The obtained residue was purified by the silica gel chromatography to obtain 1.5 g (yield: 31.0 %) of the title compound.
¹H-NMR (DMSO-d₆) δ:3.76 (3H, s), 5.11 (2H, s), 6.96 (2H, d, J=8Hz), 7.25-7.30 (1H, m), 7.33 (1H, ddd, J=8Hz, 8Hz, 1Hz), 7.73 (2H, br-s), 7.90 (1H, dd , J=8Hz, 1Hz), 8.48 (1H, d, J=8Hz), 10.38 (1H, br-s)
EI-MS (m/z, %): 412 (m+, 6), 370 (6), 292 (3), 279 (10), 121 (100)
IR (ν, cm⁻¹, KBr):1676, 1612, 1586, 1538, 1518, 1152
m. p.:164-165°C

### Reference Example 29: N-[2-[3-(4-Methoxybenzyloxy)benzamido] benzenesulfonyl]acetamide:

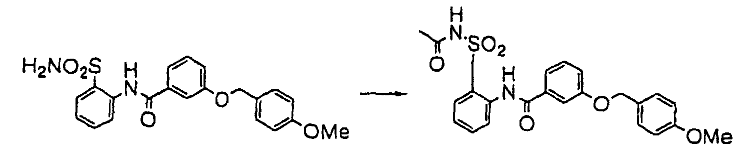

0.1 ml (1.10 mmol) of acetic acid anhydride was added to a solution of 400 mg (1.00 mmol) of 3-(4-methoxybenzyloxy)-N-(2-sulfamoylphenyl)benzamide prepared in Reference Example 28 and 237 mg (1.90 mmol) of 4-dimethylaminopyridine in anhydrous tetrahydrofuran (10 ml) at 0°C in nitrogen stream. The obtained mixture was stirred at room temperature for 1 hour and then the solvent was evaporated under reduced pressure. The residue was dissolved in ethyl acetate and then the obtained solution was washed with water, an aqueous potassium hydrogensulfate solution and saturated aqueous sodium chloride solution in that order. After drying over anhydrous sodium sulfate, the solvent was evaporated under reduced pressure. The residue was purified by the silica gel column chromatography to obtain 430 mg (yield: 97.6 %) of the title compound.
¹H-NMR (CDCl₃) δ:2.04 (3H, s), 3.82 (3H, s) , 5.07 (2H, s), 6.90-6.96 (2H, m), 7.16 (1H, ddd, J=8Hz, 2Hz, 1Hz), 7.22-7.2 9 (1H, m), 7.35-7.43 (3H, m), 7.58-7.63 (1H, m), 7.6 5-7.71 (2H, m), 7.98 (1H, dd, J = 8Hz, 1Hz), 8.27 (1H, br-s), 8.73 (1H, dd, J=8Hz, 1Hz), 10.34 (1H, br-s)
IR (ν, cm⁻¹, KBr):3384, 3084, 2872, 1718, 1658, 1612, 1580, 1538, 1518, 1476, 1448 EI-MS (m/z, %):454 (7), 334 (37), 240 (9), 121 (100)
m. p.:114-117°C

### Reference Example 30: N-[2-[3-(4-Methoxybenzyloxy)benzamido] benzenesulfonyl]hexanamide:

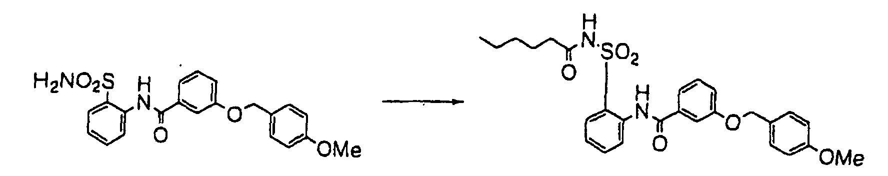

0.15 mg (1.10 mmol) of hexanoyl chloride was added to a solution of 400 mg (1.00 mmol) of 3-(4-methoxybenzyloxy)-N-(2-sulfamoylphenyl)benzamide prepared in Reference Example 28 and 237 mg (1.90 mmol) of 4-dimethylaminopyridine in anhydrous tetrahydrofuran (10 ml) at 0°C in nitrogen stream. The obtained mixture was stirred at room temperature for 1 hour and then the solvent was evaporated under reduced pressure. The residue was dissolved in ethyl acetate and then the obtained solution was washed with water, an aqueous potassium hydrogensulfate solution and saturated aqueous sodium chloride solution in that order. After drying over anhydrous sodium sulfate, the solvent was evaporated under reduced pressure. The residue was purified by the silica gel column chromatography to obtain 490 mg (yield: 98.8 %) of the title compound.
¹H-NMR (CDCl₃) δ:0.81 (3H, t, J=7Hz), 1.13-1.27 (4H, m), 1.48-1.59 (2H, m), 2.21 (2H, t, J=7Hz), 3.82 (3H, s), 5.07 (2H, s), 6.90-6.96 (2H, m), 7.16 (1H, ddd , J=8Hz, 2H z, 1Hz), 7.22-7.29 (1H, m), 7.36-7.44 (3H, m), 7.60-7.72 (3H, m), 7.99 (1H, dd, J=8Hz, 1Hz), 8.13 (1H, br-s), 8.73 (1H, dd, J = 8H z, 1Hz), 10.42 (1H, br-s)
IR (ν, cm⁻¹, KBr):3392, 3072, 2956, 2932, 2872, 1716, 1658, 1614, 1580, 1538, 1520, 1450, 1412.
EI-MS (m/z, %) : 510 (2), 390 (2), 178 (6), 154 (2), 121 (100)
m. p.:154-157°C

### Reference Example 31: N-[2-[3-(4-Methoxybenzyloxy)benzamido] benzene sulfonyl]decanamide:

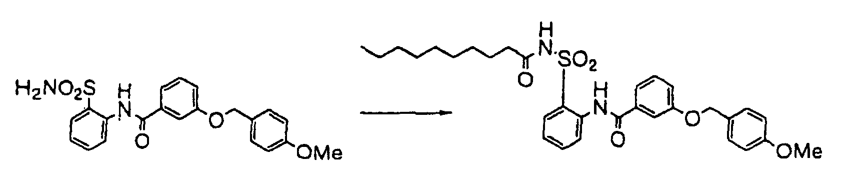

0.22 ml (1.10 mmol) of decanoyl chloride was added to a solution of 400 mg (1.00 mmol) of 3-(4-methoxybenzyloxy)-N-(2-sulfamoylphenyl)benzamide prepared in Reference Example 28 and 237 mg (1.90 mmol) of 4-dimethylaminopyridine in anhydrous tetrahydrofuran (10 ml) at 0°C in nitrogen stream. The obtained mixture was stirred at room temperature for 1 hour and then the solvent was evaporated under reduced pressure. The residue was dissolved in ethyl acetate and then the obtained solution was washed with water, an aqueous potassium hydrogensulfate solution and saturated aqueous sodium chloride solution in that order. After drying over anhydrous sodium sulfate, the solvent was evaporated under reduced pressure. The residue was purified by the silica gel column chromatography to obtain 520 mg (yield: 94.6 %) of the title compound.
¹H-NMR (CDCl₃) δ:0. 87 (3H, t, J=7Hz), 1.15-1.32 (12H, m), 1.49-1.60 (2H, m), 2.22 (2H, t, J=7Hz), 3.83 (3H, s), 5.08 (2H, s), 6.91-6.96 (2H, m), 7.17 (1H, ddd, J=8Hz, 2H z, 1Hz), 7.23-7.30 (1H, m), 7.36-7.44 (3H, m), 7.61-7.73 (3H, m), 8.00 (1H, dd, J=8Hz, 1Hz), 8.05 (1H, br-s), 8.74 (1H, dd, J = 8H z, 1Hz),10. 43 (1H, br-s)
IR (ν, cm⁻¹, KBr) : 3364, 3036, 2920, 2852, 1704, 1660, 1602, 1586, 1540, 1518, 1490, 1472, 1448, 1440.
EI-MS (m/z, %):566 (3), 275 (9), 211 (8), 121 (100)
m. p.:148-149°C

### Reference Example 32: 3-Cyclohexylmethoxy-N-(2-sulfamoylphenyl) benzamide:

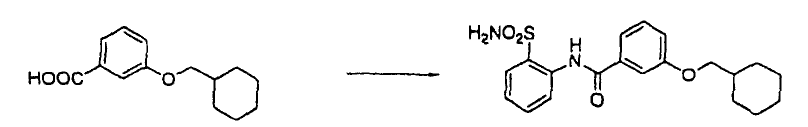

A solution of 3.00 g (12.8 mmol) of 3-cyclohexylmethoxybenzoic acid and 3 ml of thionyl chloride in benzene (30 ml) was heated under reflux for 2 hours. The solvent was evaporated under reduced pressure. The residue was dissolved in methylene chloride (30 ml). The obtained solution was added dropwise to a solution of 2.43 g (14.1 mmol) of 2-aminobenzenesulfonamide in pyridine (50 ml) under cooling with ice. The obtained mixture was stirred at room temperature for 18 hours. Methylene chloride was evaporated under reduced pressure. The residue was dissolved in ethyl acetate and then the obtained solution was washed with 1 N aqueous hydrochloric acid solution, water and saturated aqueous sodium chloride solution in that order. After drying over anhydrous sodium sulfate, the solvent was evaporated under reduced pressure. The obtained crude crystals were recrystallized from acetonitrile to obtain 2.80 g (yield: 56.0 %) of the title compound.
¹H-NMR(CDCl₃) δ:1.00-1.39 (5H, m), 1.68 -1.90 (6H, m), 3.82 (2H, d, J=7Hz), 4.95 (2H, s), 7.10 (1H, ddd, J=8Hz, 2Hz, 1Hz), 7.25 (1H, ddd, J=8Hz, 8Hz, 1Hz), 7.38 (1H, dd, J=8Hz, 8Hz), 7.45-7.50 (2H, m), 7.62 (1H, ddd, J=8Hz, 8Hz, 2Hz), 7.96 (1H, dd, J=8H z , 2Hz), 8.53 (1H, dd , J=8Hz, 1Hz), 10.03 (1H, s)
EI-MS (m/z, %):388 (m+, 44), 217 (86), 212 (95), 121 (100)
IR (ν, cm⁻¹, KBr) : 1670, 1580, 1538, 1450, 1158, 1522, 1034
m. p. : 161-162°C

### Reference Example 33: N-[2-(3-Cyclohexylmethoxybenzamido) benzenesulfonyl]acetamide:

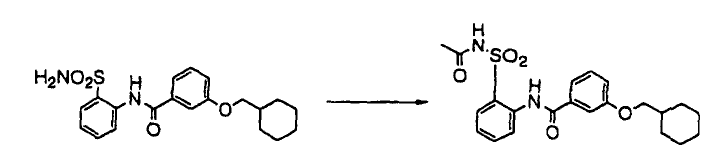

0.11 ml (1.10 mmol) of acetic acid anhydride was added to a solution of 400 mg (1.00 mmol) of 3-cyclohexylmethoxy-N-(2-sulfamoylphenyl)benzamide prepared in Reference Example 32 and 237 mg (1.90 mmol) of 4-dimethylaminopyridine in anhydrous tetrahydrofuran (10 ml) at 0°C in nitrogen stream. The obtained mixture was stirred at room temperature for 1 hour and then the solvent was evaporated under reduced pressure. The residue was dissolved in ethyl acetate and then the obtained solution was washed with water, an aqueous potassium hydrogensulfate solution and saturated aqueous sodium chloride solution in that order. After drying over anhydrous sodium sulfate, the solvent was evaporated under reduced pressure. The residue was purified by the silica gel column chromatography to obtain 430 mg (yield: 96.9 %) of the title compound.
¹H-NMR (CDCl₃) δ:1.00-1.13 (2H, m), 1.14 -1.37 (3H, m), 1.66-1.92 (6H, m), 2.06 (3H, s), 3.83 (2H, d, J=6Hz), 7.07-7.12 (1H, m), 7.22-7.29 (1H, m), 7.35-7.42 (1H, m), 7.54-7.60 (2H, m), 7.65-7.72 (1H, m), 7.98 (1H, dd, J=8Hz, 1Hz), 8.30 (1H, br-s), 8.73
(1H, dd, J=8Hz, 1Hz), 10.34 (1H, br-s)
IR (ν, cm⁻¹, KBr):3368, 3036, 2932, 2856, 1712, 1662, 1604, 1586, 1542, 1492, 1472, 1450, 1440.
EI-MS (m/z, %):430 (37), 308 (12), 275 (14), 183 (8), 121 (100)
m. p.:164-167°C

### Reference Example 34: N-[2-(3-cyclohexylmethoxybenzamido) benzenesulfonyl]hexanamide:

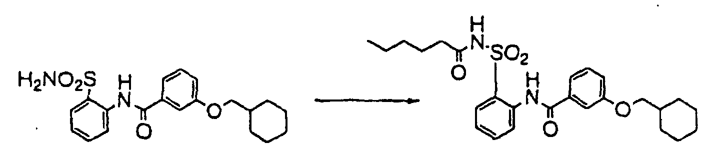

0.16 ml (1.10 mmol) of hexanoyl chloride was added to a solution of 400 mg (1.00 mmol) of 3-cyclohexylmethoxy-N-(2-sulfamoylphenyl)benzamide prepared in Reference Example 32 and 237 mg (1.90 mmol) of 4-dimethylaminopyridine in anhydrous tetrahydrofuran (10 ml) at 0°C in nitrogen stream. The obtained mixture was stirred at room temperature for 1 hour and then the solvent was evaporated under reduced pressure. The residue was dissolved in ethyl acetate and then the obtained solution was washed with water, an aqueous potassium hydrogensulfate solution and saturated aqueous sodium chloride solution in that order. After drying over anhydrous sodium sulfate, the solvent was evaporated under reduced pressure. The residue was purified by the silica gel column chromatography to obtain 490 mg (yield: 97.8 %) of the title compound.
¹H-NMR (CDCl₃) δ:0.84 (3H, t, J=7Hz), 1.0 0-1.13 (2H, m), 1.14-1.38 (7H, m), 1.50-1.61 (2H, m), 1.66-1.92 (6H, m), 2.23 (2H, t, J=7Hz), 3.83 (2H, d, J=6Hz), 7.07-7.12 (1 H, m), 7.22-7.29 (1H, m), 7.36-7.42 (1H, m), 7.55-7.61 (2H, m), 7.65-7.71 (1H, m), 7.99 (1H, dd , J=8Hz, 1Hz), 8.11 (1H,br-s), 8.73 (1H, dd, J=8Hz, 1Hz), 10. 40 (1H,br-s) IR (ν, cm⁻¹, KBr):3380, 3080, 2924, 2856, 1714, 1690, 1664, 1580, 1538, 1476, 1448, 1406.
EI-MS (m/z, %):486 (46), 275 (14), 183 (8), 121 (100)
m. p.:112-113°C

### Reference Example 35: N-[2-(3-Cyclohexylmethoxybenzamido) benzenesulfonyl]decanamide:

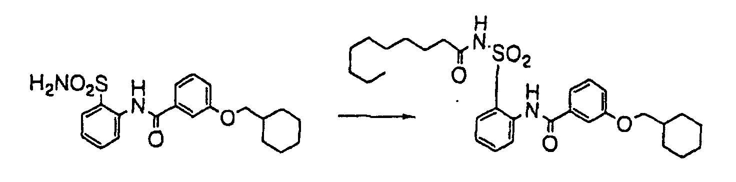

0.24 ml (1.10 mmol) of decanoyl chloride was added to a solution of 400 mg (1.00 mmol) of 3-cyclohexylmethoxy-N-(2-sulfamoylphenyl)benzamide prepared in Reference Example 32 and 237 mg (1.90 mmol) of 4-dimethylaminopyridine in anhydrous tetrahydrofuran (10 ml) at 0°C in nitrogen stream. The obtained mixture was stirred at room temperature for 1 hour and then the solvent was evaporated under reduced pressure. The residue was dissolved in ethyl acetate and then the obtained solution was washed with water, an aqueous potassium hydrogensulfate solution and saturated aqueous sodium chloride solution in that order. After drying over anhydrous sodium sulfate, the solvent was evaporated under reduced pressure. The residue was purified by the silica gel column chromatography to obtain 550 mg (yield: 98.4 %) of the title compound.
¹H-NMR(CDCl₃) δ:0.87 (3H, t, J=7Hz), 1.01-1.13 (2H, m), 1.15-1.37 (15H, m), 1.49-1.59 (2H, m), 1.66-1.92 (6H, m), 2.23 (2H, t, J=7Hz), 3.83 (2H, d, J=6Hz), 7.07-7.12 (1H, m), 7.22-7.29 (1H, m), 7.36-7.42 (1H, m), 7.55-7.62 (2H, m), 7.64-7.72 (1H, m), 7.99 (1H, dd , J = 8Hz, J=1Hz), 8.08 (1H,br -s), 8.73 (1H, dd, J=8Hz, 1Hz), 10.40 (1H, br-s)
IR (ν, cm⁻¹, KBr):3360, 3028, 2924, 2856, 1708,
1658, 1540, 1472, 1448.
EI-MS (m/z, %):542 (29), 275 (9), 183 (6), 121 (100)
m. p.:124-127°C

### Reference Example 36: N-[2-[3-(4-t-Butylbenzyloxy)benzamido] benzenesulfonyl]acetamide:

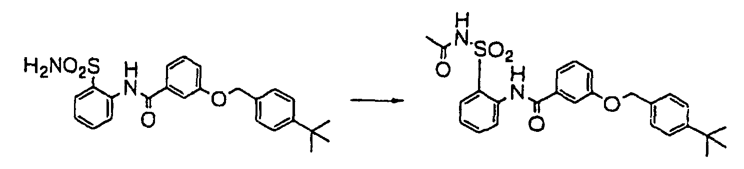

0.11 ml (1.10 mmol) of acetic acid anhydride was added to a solution of 438 mg (1.00 mmol) of 3-(4-t-butylbenzyloxy)-N-(2-sulfamoylphenyl)benzamide prepared in Reference Example 13 and 249 mg (2.00 mmol) of 4-dimethylaminopyridine in anhydrous tetrahydrofuran (10 ml) at 0°C in nitrogen stream. The obtained mixture was stirred at room temperature for 1 hour and then the solvent was evaporated under reduced pressure. The residue was dissolved in ethyl acetate and then the obtained solution was washed with water, an aqueous potassium hydrogensulfate solution and saturated aqueous sodium chloride solution in that order. After drying over anhydrous sodium sulfate, the solvent was evaporated under reduced pressure. The residue was purified by the silica gel column chromatography to obtain 456 mg (yield: 96.0 %) of the title compound.
¹H-NMR (δ. CDCl₃):1.29 (9H, s), 1.93 (3H, s
), 5.15 (2H, s), 7.17 (1H, ddd, J=8Hz, 2Hz, 1Hz), 7.22-7.29 (1H, m), 7.38-7.44 (5H, m), 7.60-7.63 (1H, m), 7.65-7.71 (2H, m), 7.99 (1H, dd, J=8Hz, 2Hz) 8.73 (1H, dd, J=8Hz, 1Hz), 7.56 (1H, d, J = 7Hz), 7.60 (1H, s), 7.72 (1H, m), 7.91 (1H, d, J=8Hz), 8.40 (1H, d, J=8Hz), 10.40 (1H, br-s)
EI-MS (m/z,):480 (m+, 26), 422 (1), 267 (4), 147 (100)
IR (ν, cm⁻¹, KBr):3384, 2956, 2868, 1714, 1658, 1580, 1538
m.p.:190-191°C

### Reference Example 37: N-[2-[3-(4-t-Butylbenzyloxy)benzamido] benzenesulfonyl]hexanamide:

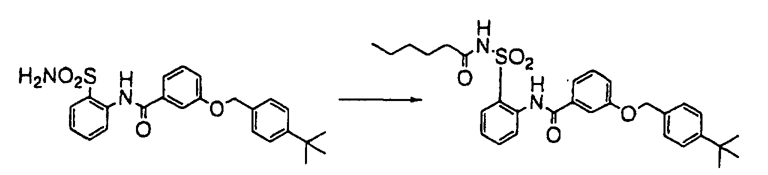

0.16 ml (1.10 mmol) of hexanoyl chloride was added to a solution of 438 mg (1.00 mmol) of 3-(4-t-butylbenzyloxy)-N-(2-sulfamoylphenyl)benzamide prepared in Reference Example 13 and 249 mg (2.00 mmol) of 4-dimethylaminopyridine in anhydrous tetrahydrofuran (10 ml) at 0°C in nitrogen stream. The obtained mixture was stirred at room temperature for 1 hour and then the solvent was evaporated under reduced pressure. The residue was dissolved in ethyl acetate and then the obtained solution was washed with water, an aqueous potassium hydrogensulfate solution and saturated aqueous sodium chloride solution in that order. After drying over anhydrous sodium sulfate, the solvent was evaporated under reduced pressure. The residue was purified by the silica gel column chromatography to obtain 488 mg (yield: 91.0 %) of the title compound.
¹H-NMR (δ, CDCl₃):0.82 (3H, t , J=7Hz), 1.2
1 (4H, m), 1.33 (9H, s), 1.54 (2H, m), 2.23 (2H, t, J=7Hz), 5.10 (2H, s), 7.18 (1H, ddd, J =8Hz, 2Hz, 1Hz), 7.26 (1H, ddd, J=8Hz, 8H z, 2Hz), 7.38-7.44 (5H, m), 7.62 (1H, d, J= 8Hz), 7.65-7.74 (2H, m), 7.96 (1H, br-s), 8.00 (1H, dd , J=8Hz, 2Hz), 8.74 (1H, dd , J =8Hz. 1Hz), 10.43 (1H, br-s)
EI-MS (m/z,):536 (m+, 49), 422 (4), 267 (14), 147 (100), 91 (21), 71 (4)
IR (ν, c m⁻¹, KBr):3368, 2960, 2868, 1702, 1662, 1586, 1538
m. p.:163-164°C

### Reference Example 38: N-[2-[3-(4-t-Butylbenzyloxy)benzamido] benzenesulfonyl]decanamide:

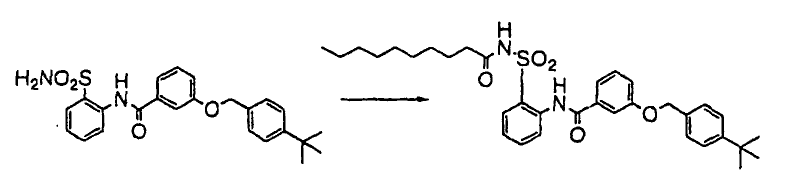

0.24 ml (1.10 mmol) of decanoyl chloride was added to a solution of 438 mg (1.00 mmol) of 3-(4-t-butylbenzyloxy)-N-(2-sulfamoylphenyl)benzamide prepared in Reference Example 13 and 249 mg (2.00 mmol) of 4-dimethylaminopyridine in anhydrous tetrahydrofuran (10 ml) at 0°C in nitrogen stream. The obtained mixture was stirred at room temperature for 1 hour and then the solvent was evaporated under reduced pressure. The residue was dissolved in ethyl acetate and then the obtained solution was washed with water, an aqueous potassium hydrogensulfate solution and saturated aqueous sodium chloride solution in that order. After drying over anhydrous sodium sulfate, the solvent was evaporated under reduced pressure. The residue was purified by the silica gel column chromatography to obtain 535 mg (yield: 99.0 %) of the title compound.
¹H-NMR (δ. CDCl₃):0.86 (3H, t, J=7Hz), 1.22 (14H, m), 1.33 (9H, s), 2.23 (2H, t, J=7Hz), 5.10 (2H, s), 7.18 (1H, ddd, J=8Hz, 2Hz, 1Hz), 7.26 (1H, ddd, J=8Hz, 8Hz, 2Hz), 7.38 -7.44 (5H, m), 7.62 (1H, d, J=8Hz), 7.65-7.74 (2H, m), 7.94 (1H, br-s), 8.00(1H, dd, J =8Hz, 2Hz), 8.74 (1H, dd, J=8Hz, 1Hz), 10.43 (1H, br-s)
EI-MS (m/z,):592 (m+, 30), 422 (3), 267 (16), 147 (100)
IR (ν, cm⁻¹, KBr):3368, 3064, 3036, 2960, 2924, 2856, 1704, 1660, 1586, 1540
m. p. : 132-133°C

### Reference Example 39: 3-(4-Trifluoromethylbenzyloxy)-N-(2-sulfamoylphenyl)benzamide:

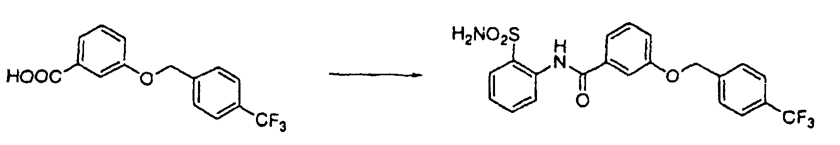

A solution of 4.00 g (14.1 mmol) of 3-(4-trifluoromethylbenzyloxy)benzoic acid and 3 ml of thionyl chloride in benzene (30 ml) was heated under reflux for 2 hours. The solvent was evaporated under reduced pressure. The residue was dissolved in methylene chloride (30 ml). The obtained solution was addeddropwise to a solution of 2.66 g (15.4 mmol) of 2-aminobenzenesulfonamide in pyridine (50 ml) under cooling with ice. The obtained mixture was stirred at room temperature for 18 hours. Methylene chloride was evaporated under reduced pressure. The residue was dissolved in ethyl acetate and then the obtained solution was washed with 1 N aqueous hydrochloric acid solution, water and saturated aqueous sodium chloride solution in that order. After drying over anhydrous sodium sulfate, the solvent was evaporated under reduced pressure. The obtained crude crystals were washed with methanol to obtain 5.10 g (yield: 80.0 %) of the title compound.
¹H-NMR (DMSO-d₆) δ:5.33 (2H, s), 729-7.36 (2H, m), 7.50-7.58 (3H, m), 7.65 (1H, ddd , J=8Hz, 8Hz, 2Hz), 7.69 (2H, d, J=8Hz), 7.78 (2H, d, J=8Hz), 7.90 (1H, dd, J=8Hz, 2Hz), 8.47 (1H, dd, J=8Hz, 1Hz)
EI-MS (m/z, %):450 (m+, 68), 371 (100), 279 (100), 159 (100)
IR (ν, cm⁻¹, KBr):1678, 1586, 1538, 1326, 1066
m. p.:169-170°C

### Reference Example 40: N-[2-[3-(4-Trifluoromethylbenzyloxy) benzamido]benzenesulfonyl]acetamide:

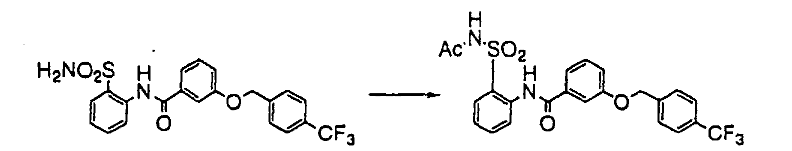

0.11 ml (1.10 mmol) of acetic acid anhydride was added to a solution of 451 mg (1.00 mmol) of 3-(4-trifluoromethylbenzyloxy)-N-(2-sulfamoylphenyl)benzamide prepared in Reference Example 39 and 249 mg (2.00 mmol) of 4-dimethylaminopyridine in anhydrous tetrahydrofuran (10 ml) at 0°C in nitrogen stream. The obtained mixture was stirred at room temperature for 1 hour and then the solvent was evaporated under reduced pressure. The residue was dissolved in ethyl acetate and then the obtained solution was washed with water, an aqueous potassium hydrogensulfate solution and saturated aqueous sodium chloride solution in that order. After drying over anhydrous sodium sulfate, the solvent was evaporated under reduced pressure. The residue was purified by the silica gel column chromatography to obtain 476 mg (yield: 96.7 %) of the title compound.
¹H-NMR (CDCl₃) δ:2.05 (3H, s), 5.21 (2H, s) , 7.16 (1H, ddd, J=8Hz, J=2Hz, J=1Hz), 7.26 (1H, ddd, J=8Hz, 8Hz, 2Hz), 7.42 (1H, d d, J=8Hz, 8Hz), 7.57 (2H, d, J=8Hz), 7.65-7.74 (5H, m), 7.96 (1H, dd, J=8Hz, 2Hz), 8.30 (1H, br-s), 8.73 (1H, dd, J=8Hz, 1Hz), 10.44 (1H, br-s)
EI-MS (m/ z,):492 (m+, 3), 370 (35), 279 (92), 159 (100), 121 (22)
IR (ν, cm⁻¹, KBr):3372, 3124, 2880, 1724, 1678, 1614, 1588, 1546
m. p. : 164-165°C

### Reference Example 41: N-[2-[3-(4-Trifluoromethylbenzyloxy)benzamido] benzenesulfonyl]hexanamide:

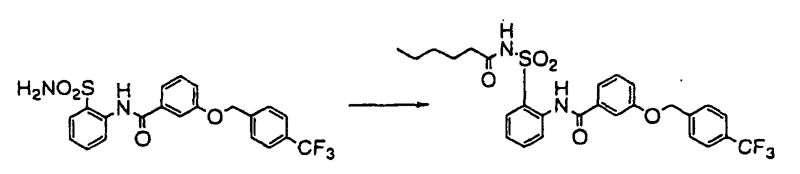

0.16 ml (1.10 mmol) of hexanoyl chloride was added to a solution of 451 mg (1.00 mmol) of 3-(4-trifluoromethylbenzyloxy)-N-(2-sulfamoylphenyl)benzamide prepared in Reference Example 39 and 249 mg (2.00 mmol) of 4-dimethylaminopyridine in anhydrous tetrahydrofuran (10 ml) at 0°C in nitrogen stream. The obtained mixture was stirred at room temperature for 1 hour and then the solvent was evaporated under reduced pressure. The residue was dissolved in ethyl acetate and then the obtained solution was washed with water, an aqueous potassium hydrogensulfate solution and saturated aqueous sodium chloride solution in that order. After drying over anhydrous sodium sulfate, the solvent was evaporated under reduced pressure. The residue was purified by the silica gel chromatography to obtain 439 mg (yield: 80.0 %) of the title compound.
¹H-NMR (CDCl₃) δ:0. 82 (3H, t, J=7Hz), 1.21 (4H, m), 1.55 (2H, m), 2.23 (2H, t, J=7Hz), 5.21 (2H, s), 7.17 (1H, ddd, J=8Hz, 2Hz, 1Hz), 7.26 (1H, ddd, J=8Hz, 8Hz, 2Hz), 7.43 (1H, dd, J=8Hz, 8Hz), 7.57 (2H, d, J=8Hz), 7.63-7.72 (5H, m), 7.98 (1H, dd, J=8Hz, 2Hz), 8.02 (1H, br-s), 8.74 (1H, dd, J=8Hz, 1Hz), 10. 46 (1H, br-s)
EI-MS (m/ z,):548 (m+, 25), 370 (50), 279 (100), 159 (96), 121 (25)
IR (ν, cm⁻¹, KBr) : 3380, 3080, 2960, 2932, 2872, 1708, 1660, 1584, 1540
m. p. : 142-143°C

### Reference Example 42: N-[2-[3-(4-Trifluoromethylbenzyloxy) benzamido] benzenesulfonyl]decanamide:

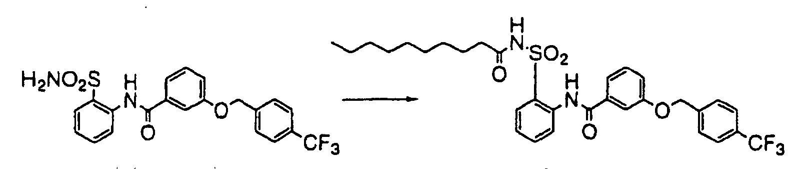

0.24 ml (1.10 mmol) of decanoyl chloride was added to a solution of 451 mg (1.00 mmol) of 3-(4-trifluoromethylbenzyloxy)-N-(2-sulfamoylphenyl)benzamide prepared in Reference Example 39 and 249 mg (2.00 mmol) of 4-dimethylaminopyridine in anhydrous tetrahydrofuran (10 ml) at 0°C in nitrogen stream. The obtained mixture was stirred at room temperature for 1 hour and then the solvent was evaporated under reduced pressure. The residue was dissolved in ethyl acetate and then the obtained solution was washed with water, an aqueous potassium hydrogensulfate solution and saturated aqueous sodium chloride solution in that order. After drying over anhydrous sodium sulfate, the solvent was evaporated under reduced pressure. The residue was purified by the silica gel column chromatography to obtain 512 mg (yield: 84.6 %) of the title compound.
¹H-NMR (CDCl₃) δ:0.86 (3H, t, 7Hz), 1.16-12.8 (12H, m), 1.53 (2H, m), 2.23 (2H, t, J = 7Hz), 5.21 (2H, s), 7.16 (1H, ddd, J=8Hz, 2Hz, 1Hz), 7.26 (1H, ddd, J=8Hz, 8Hz, 2Hz), 7.43 (1H, dd, J=8Hz, 8Hz), 7.58 (2H, d, 8H z), 7.63-7.73 (5H, m), 7.97 (1H, dd, J=8Hz, 2Hz), 8.25 (1H, br-s), 8.73 (1H, dd, J=8Hz, 1Hz), 10.47 (1H, br-s)
E I-MS (m/ z,):604 (m+, 28), 370 (57), 279 (100), 159 (82)
IR (ν, cm⁻¹, KBr):3372, 3036, 2924, 2852, 2788, 1702, 1666, 1606, 1588, 1544
m. p.: 164°C

### Reference Example 43: 3-Heptyloxy-N-(2-sulfamoylphenyl)benzamide:

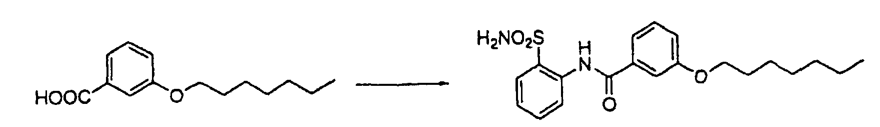

A solution of 3 g (12.5 mmol) of 3-heptyloxybenzoic acid and 3 ml of thionyl chloride in benzene (30 ml) was heated under reflux for 2 hours. The solvent was evaporated under reduced pressure. The residue was dissolved in methylene chloride (30 ml). The obtained solution was addeddropwise to a solution of 2.50 g (14.5 mmol) of 2-aminobenzenesulfonamide in pyridine (50 ml) under cooling with ice. The obtained mixture was stirred at room temperature for 18 hours. Methylene chloride was evaporated under reduced pressure. The residue was dissolved in ethyl acetate and then the obtained solution was washed with 1 N aqueous hydrochloric acid solution, water and saturated aqueous sodium chloride solution in that order. After drying over anhydrous sodium sulfate, the solvent was evaporated under reduced pressure. The obtained crude crystals were recrystallized from acetonitrile to obtain 3.00 g (yield: 60.0 %) of the title compound.
¹H-NMR (DMSO-d₆) δ:0.87 (3H, t, J=7Hz), 1.22-1.50 (8H, m), 1.70-1.80 (2H, m), 4.41 (2H, t, J=7Hz), 7.17-7.23 (1H, m), 7.33 (1H, ddd, J=8Hz, 8Hz, 2Hz), 7.77 (2H, s). 7.90 (1 H, dd, J=8Hz, 2Hz), 8.4 8 (1H, d, J=8Hz), 10.38 (1H, s)
EI-MS (m/z, %):390 (m+, 82), 311 (43), 310 (100), 219 (100)
IR (ν, cm⁻¹, KBr):1674, 1612, 1586, 1544, 1336, 1140
m. p.: 112-113°C

### Reference Example 44: N-[2-(3-Heptyloxybenzamido) benzenesulfonyl] acetamide:

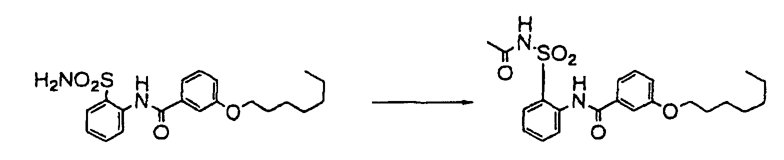

0.11 ml (1.10 mmol) of acetic acid anhydride was added to a solution of 391 mg (1.00 mmol) of 3-heptyloxy-N-(2-sulfamoylphenyl)benzamide prepared in Reference Example 43 and 249 mg (2.00 mmol) of 4-dimethylaminopyridine in anhydrous tetrahydrofuran (10 ml) at 0°C in nitrogen stream. The obtained mixture was stirred at room temperature for 1 hour and then the solvent was evaporated under reduced pressure. The residue was dissolved in ethyl acetate and then the obtained solution was washed with water, an aqueous potassium hydrogensulfate solution and saturated aqueous sodium chloride solution in that order. After drying over anhydrous sodium sulfate, the solvent was evaporated under reduced pressure. The residue was purified by the silica gel column chromatography to obtain 411 mg (yield: 95.0 %) of the title compound.
¹H-NMR (DMSO-d₆) δ:0.87 (3H, t, J=7Hz), 1.35 (8H, m), 1.68 (3H, s), 1.75 (2H, m), 4.04 (2H, t, J=6Hz), 7.10-7.15 (2H, m), 7.38-7. 46 (2H, m), 7.57-7.64 (2H, m), 7.75 (1H, dd, J=8Hz, 2Hz), 8.34 (1H, dd, J=8Hz, 1Hz), 11.51 (1H, s)
EI-MS (m/ z,) : 432 (m+, 7), 310 (31), 219 (100), 121 (12)
IR (ν, cm⁻¹, KBr):2928, 2856, 1684, 1586, 1552, 1494
m. p.:194-195°C

### Reference Example 45: N-[2-(3-Heptyloxybenzamido) benzenesulfonyl] hexan amide:

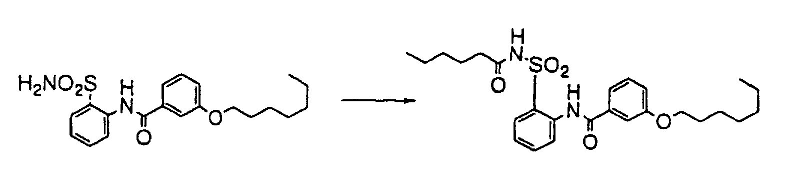

0.16 ml (1.10 mmol) of hexanoyl chloride was added to a solution of 390 mg (1.00 mmol) of 3-heptyloxy-N-(2-sulfamoylphenyl)benzamide prepared in Reference Example 43 and 249 mg (2.00 mmol) of 4-dimethylaminopyridine in anhydrous tetrahydrofuran (10 ml) at 0°C in nitrogen stream. The obtained mixture was stirred at room temperature for 1 hour and then the solvent was evaporated under reduced pressure. The residue was dissolved in ethyl acetate and then the obtained solution was washed with water, an aqueous potassium hydrogensulfate solution and saturated aqueous sodium chloride solution in that order. After drying over anhydrous sodium sulfate, the solvent was evaporated under reduced pressure. The residue was purified by the silica gel column chromatography to obtain 412 mg (yield: 84.4 %) of the title compound.
¹H-NMR (DMSO-d₆) δ:0.76 (3H, t, J=7Hz), 0.87 (3H, d, J=7Hz) 1.12 (4H, m), 1.26-1.47 (10H, m), 1.75 (2H, m), 1.92 (2H, m), 4.03 (2 H, t, J=6Hz), 7.10-7.15 (2H, m), 7.38-7.46 (2H, m), 7.5 8 (1H, ddd, J=8Hz, 2Hz, 1Hz), 7.65 (1H, dd, J=2Hz, 1Hz) 7.74 (1H, dd, J= 8Hz, 2Hz), 8.34 (1H, dd, J=8Hz, 1Hz), 11.46 (1H, s) EI-MS (m/ z,):488 (m+, 2), 310 (12), 219 (69), 196 (100), 121 (40)
IR (ν, cm⁻¹, KBr):2928, 2860, 1684, 1594, 1552, 1494
m. p.:200-201°C

### Reference Example 46: N-[2-(3-Heptyloxybenzamido)benzenesulfonyl] hexanamide:

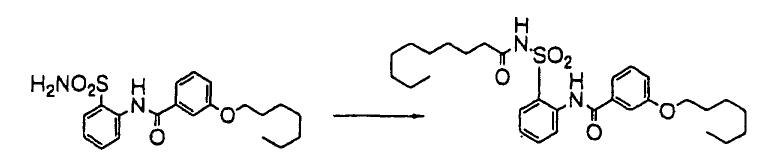

0.24 ml (1.10 mmol) of decanoyl chloride was added to a solution of 390 mg (1.00 mmol) of 3-heptyloxy-N-(2-sulfamoylphenyl)benzamide prepared in Reference Example 43 and 249 mg (2.00 mmol) of 4-dimethylaminopyridine in anhydrous tetrahydrofuran (10 ml) at 0°C in nitrogen stream. The obtained mixture was stirred at room temperature for 1 hour and then the solvent was evaporated under reduced pressure. The residue was dissolved in ethyl acetate and then the obtained solution was washed with water, an aqueous potassium hydrogensulfate solution and saturated aqueous sodium chloride solution in that order. After drying over anhydrous sodium sulfate, the solvent was evaporated under reduced pressure. The residue was purified by the silica gel column chromatography to obtain 495 mg (yield: 91.0 %) of the title compound.
¹H-NMR(DMSO-d₆) δ:0.84 (6H, m), 1.04-1.47 (2H, m), 1.75 (2H, m), 1.91 (2H, t, J=7Hz), 4.04 (2H, t, J=6Hz), 7.10-7.15 (2H, m), 7.38-7.46 (2H, m), 7.58 (1H, d, J=8Hz), 7.66 (1H, dd, J=2Hz, 1Hz) 7.74 (1H, dd, J=8Hz, 2Hz), 8.34 (1H, dd , J=8Hz, 1Hz), 11.43 (1H, s)
EI-MS (m/ z,):544 (in+, 2), 310 (21), 219 (100), 121 (36)
IR (ν, cm⁻¹, KBr) : 2928, 2856, 1684, 1592, 1552, 1494
m. p.: 172-174°C

### Reference Example 47: 4-Phenylethynyl-N-(2-sulfamoylphenyl) benzamide:

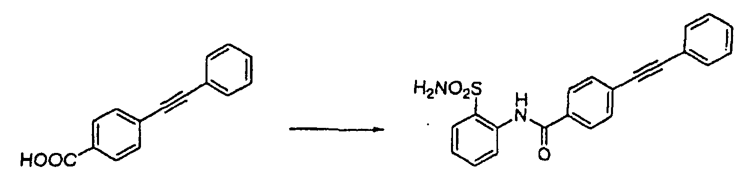

A solution of 3.00 g (13.5 mmol) of 4-phenylethynylbenzoic acid and 2 ml of thionyl chloride in benzene (30 ml) was heated under reflux for 2 hours. The solvent was evaporated under reduced pressure. The residue was dissolved in methylene chloride (30 ml). The obtained solution was addeddropwise to a solution of 2.32 g (13.50 mmol) of 2-aminobenzenesulfonamide in pyridine (50 ml) under cooling with ice. The obtained mixture was stirred at room temperature for 18 hours. Methylene chloride was evaporated under reduced pressure. The residue was dissolved in ethyl acetate and then the obtained solution was washed with 1 N aqueous hydrochloric acid solution, water and saturated aqueous sodium chloride solution in that order. After drying over anhydrous sodium sulfate, the solvent was evaporated under reduced pressure. The residue was recrystallized from ethyl acetate - hexane to obtain 4.00 g (yield: 78.7 %) of the title compound.
¹H-NMR (DMSO-d₆) δ:7.34-7.39 (1H, m), 7.44-7.50 (3H, m), 7.58-7.70 (3H, m), 7.77 (2 H, d, J=8Hz), 7.92 (1H, dd, J=8Hz, 1Hz), 7.97 (2H, d, J=8Hz), 8.44 (1H, d, J=8Hz), 10.50 (1H, br-s)

### Reference Example 48: N-[2-(4-Phenylethynylbenzamido) benzenesulfonyl]acetamide:

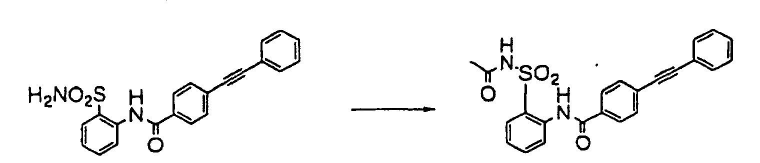

0.15 ml (1.60 mmol) of anhydrous acetic acid was added to a solution of 500 mg (1.30 mmol) of 4-phenylethynyl-N-(2-sulfamoylphenyl)benzamide prepared in Reference Example 47 and 320 mg (2.60 mmol) of 4-dimethylaminopyridine in anhydrous tetrahydrofuran (10 ml) at 0°C in nitrogen stream. The obtained mixture was stirred at room temperature for 1 hour and then the solvent was evaporated under reduced pressure. The residue was dissolved in ethyl acetate and then the obtained solution was washed with water, an aqueous potassium hydrogensulfate solution and saturated aqueous sodium chloride solution in that order. After drying over anhydrous sodium sulfate, the solvent was evaporated under reduced pressure. The residue was recrystallized from methanol to obtain 430 mg (yield: 77.6 %) of the title compound.
¹H-NMR (CDCl₃) δ:2.07 (3H, s), 7.24-7.30 (1H, m), 7.34-7.40 (3H, m), 7.53-7.60 (2H, m), 7.65 (2H, d, J=8Hz), 7.66-7.72 (1H, m), 7.99 (1H, dd, J=8, 1Hz), 8.03 (2H, d, J=8H z), 8.42 (1H, dd, J=8, 1Hz), 10.47 (1H, s)
IR (ν, cm⁻¹, KBr) : 3384, 1712, 1658, 1588, 1538, 1342, 1172, 764
EI-MS (m/ z,) : 418 (m+, 25), 296 (13), 267 (3), 205 (100), 176 (22)
m. p. : 214-215°C

### Reference Example 49 N-[2-(4-Phenylethynylbenzamido) benzenesulfonyl]hexanamide:

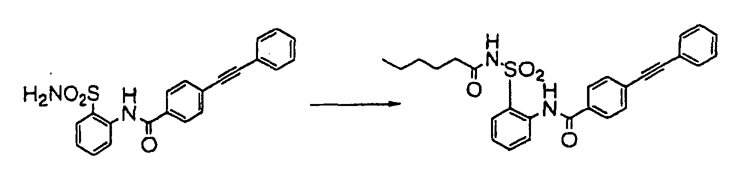

0.20 ml (1.46 mmol) of hexanoyl chloride was added to a solution of 500 mg (1.30 mmol) of 4-phenylethynyl-N-(2-sulfamoylphenyl)benzamide prepared in Reference Example 47 and 320 mg (2.60 mmol) of 4-dimethylaminopyridine in anhydrous tetrahydrofuran (10 ml) at 0°C in nitrogen stream. The obtained mixture was stirred at room temperature for 1 hour and then the solvent was evaporated under reduced pressure. The residue was dissolved in ethyl acetate and then the obtained solution was washed with water, an aqueous potassium hydrogensulfate solution and saturated aqueous sodium chloride solution in that order. After drying over anhydrous sodium sulfate, the solvent was evaporated under reduced pressure. The residue was purified by the silica gel chromatography to obtain 460 mg (yield: 74.6 %) of the title compound.
¹H-NMR (CDCl₃) δ:0.84 (3H, t, 1=7Hz), 1.16-1.30 (4H, m), 1.52-1.60 (2H, m), 2.24 (2 H, t, J=7Hz), 7.24-7.29 (1H, m), 7.35-7.40 (3H, m), 7.54-7.59 (2H, m), 7.65 (2H, d, J =8Hz), 7.66-7.72 (1H, m), 7.98 (1H, dd, J= 8Hz, 1Hz), 8.04 (2H, d, J = 8 Hz), 8.16 (H, br - s), 8.74 (1H, dd, J=8Hz, 1Hz), 10.49 (1H. s)
IR (ν, cm⁻¹, KBr):3372, 2956, 1712, 1662, 1590, 1440, 1340, 1142, 766
EI-MS (m/z,):474 (m+, 22), 376 (3), 296 (16), 267 (3), 205 (100), 176 (17)
m. p.:175-176°C

### Reference Example 50 N-[2-(4-Phenylethynylbenzamido) benzenesulfonyl]decanamide:

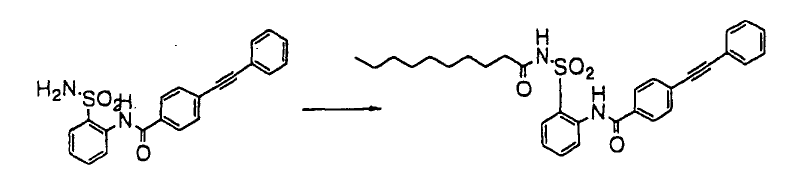

0.30 ml (1.46 mmol) of decanoyl chloride was added to a solution of 500 mg (1.30 mmol) of 4-phenylethynyl-N-(2-sulfamoylphenyl)benzamide prepared in Reference Example 47 and 320 mg (2.60 mmol) of 4-dimethylaminopyridine in anhydrous tetrahydrofuran (10 ml) in nitrogen stream. The obtained mixture was stirred at room temperature for 1 hour and then the solvent was evaporated under reduced pressure. The residue was dissolved in ethyl acetate and then the obtained solution was washed with water, an aqueous potassium hydrogensulfate solution and saturated aqueous sodium chloride solution in that order. After drying over anhydrous sodium sulfate, the solvent was evaporated under reduced pressure. The residue was recrystallized by the silica gel chromatography to obtain 540 mg (yield: 76.4 %) of the title compound.
¹H-NMR (CDCl₃) δ:0.86 (3H, t, J=7Hz), 1.18-1.32 (12H, m), 1.50-1.62 (4H, m), 2.24 (2 H, t, J=7Hz), 7.24-7.29 (1H, m), 7.35-7.40 (3H, m), 7.54-7.58 (2H, m), 7.65 (2H, d, J =8Hz), 7.66-7.72 (1H, m), 7.98 (1H, dd, J= 8Hz, 1Hz), 8.04 (2H, d, J=8Hz), 8.16 (1H,br - s), 8.74 (1H, dd, J=8Hz, 1Hz), 10.49 (1H, s)
IR (ν, cm⁻¹, KBr):3368, 2956, 1712, 1660, 1588, 1540, 1442, 1340, 1144, 764
EI-MS (m/z,):530 (m+, 21), 376 (5), 296 (18), 267 (3), 205 (100), 176 (14)
m. p.: 155-156°C

### Reference Example 51 N-[2-(4-Phenylethynylbenzamido) benzenesulfonyl]pivalamide:

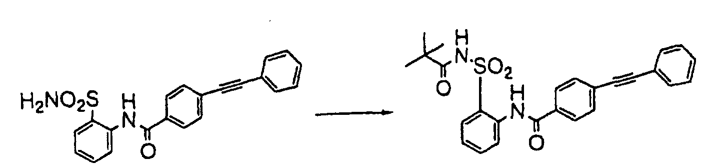

0.16 ml (1.31 mmol) of pivaloyl chloride was added to a solution of 400 mg (1.06 mmol) of 4-phenylethynyl-N-(2-sulfamoylphenyl)benzamide prepared in Reference Example 47 and 260 mg (2.12 mmol) of 4-dimethylaminopyridine in anhydrous tetrahydrofuran (10 ml) at 0°C in nitrogen stream. The obtained mixture was stirred at room temperature for 1 hour and then the solvent was evaporated under reduced pressure. The residue was dissolved in ethyl acetate and then the obtained solution was washed with water, an aqueous potassium hydrogensulfate solution and saturated aqueous sodium chloride solution in that order. After drying over anhydrous sodium sulfate, the solvent was evaporated under reduced pressure. The residue was recrystallized by the silica gel chromatography to obtain 360 mg (yield: 73.0 %) of the title compound.
¹H-NMR (CDCl₃) δ:1.15 (9H, s), 7.27 (1H, ddd, J=8Hz, 8Hz, 2Hz), 7.32-7.42 (3H, m), 7.52-7.60 (2H, m), 7.65-7.72 (3H, m), 7.97 (1 H, dd, J=8Hz, 2Hz), 8.04 (2H, dd, J=9Hz, 2 Hz), 8.23 (1H, br-s), 8.73 (1H, dd, J=8Hz, 2Hz), 10.47 (1H, s)
IR (ν, cm⁻¹, KBr):2220, 1712, 1680, 1606, 1588, 1532, 1474, 1440, 1256, 1108 EI-MS (m/z, %):460 (m+, 55), 296 (34), 205 (100), 176 (30)
m. p. : 236-237

### Reference Example 52 3-Methyl-N-[2-(4-phenylethynylbenzamido) benzenesulfonyl]-2-butenamide:

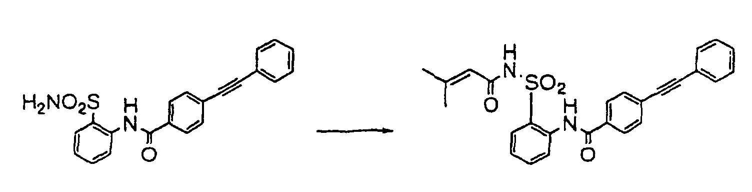

0.10 ml (0.96 mmol) of 3,3-dimethylacryloyl chloride was added to a solution of 300 mg (0.80 mmol) of 4-phenylethynyl-N-(2-sulfamoylphenyl)benzamide prepared in Reference Example 47 and 195 mg (1.60 mmol) of 4-dimethylaminopyridine in anhydrous tetrahydrofuran (10 ml) at 0°C in nitrogen stream. The obtained mixture was stirred at room temperature for 1 hour and then the solvent was evaporated under reduced pressure. The residue was dissolved in ethyl acetate and then the obtained solution was washed with water, an aqueous potassium hydrogensulfate solution and saturated aqueous sodium chloride solution in that order. After drying over anhydrous sodium sulfate, the solvent was evaporated under reduced pressure. The residue was purified by the silica gel chromatography to obtain 183 mg (yield: 50.0 %) of the title compound.
¹H-NMR(CDCl₃) δ:1.55 (3H, s), 1.88 (3H, s) , 5.51 (1H, s), 7.27 (1H, ddd, J=8Hz, 8Hz, 2H.z), 7.37-7.41 (3H, m) 7.55-7.59 (2H, m), 7.64-7.60 (3H, m), 7.93 (1H, br-s), 8.00 (1H, dd, J=8Hz, 2Hz), 8.07 (2H, dd, J=8Hz, 2Hz), 8.72 (1H, dd, J=8Hz, 2Hz), 10. 60 (1H, s)
IR (ν, cm⁻¹, KBr):2216, 1698, 1684, 1658, 1644, 1608, 1442, 1334, 1300, 1118
EI-MS (m/z, %) 458 (m+, 65), 376 (15), 296 (3 9), 205 (100), 176 (52)
m. p.:187-188°C

### Reference Example 53: Trans-N-[2-(4-phenylethynylbenzamido) benzenesulfonyl]-2,4-hexadienamide:

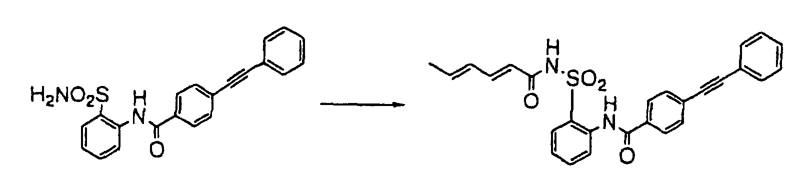

0.10 ml (0.96 mmol) of 2,4-hexadienoyl chloride was added to a solution of 300 mg (0.80 mmol) of 4-phenylethynyl-N-(2-sulfamoylphenyl)benzamide prepared in Reference Example 47 and 195 mg (1.60 mmol) of 4-dimethylaminopyridine in anhydrous tetrahydrofuran (10 ml) at 0°C in nitrogen stream. The obtained mixture was stirred at room temperature for 1 hour and then the solvent was evaporated under reduced pressure. The residue was dissolved in ethyl acetate and then the obtained solution was washed with water, an aqueous potassium hydrogensulfate solution and saturated aqueous sodium chloride solution in that order. After drying over anhydrous sodium sulfate, the solvent was evaporated under reduced pressure. The obtained crude crystals were recrystallized from methanol to obtain 180 mg (yield: 48.0 %) of the title compound.
¹H-NMR (CDCl₃) δ:1.86 (3H, d, J=6Hz), 5.65 (1H, d, J=15Hz), 6.10-6.26 (2H, m), 7.22 -7.3 0 (2H, m), 7.34-7.40 (3H, m), 7.54-7.59 (2H, m), 7.64-7.70 (3H, m), 7.99 (1H, dd, J=8Hz, 2 Hz), 8.02 (1H, br-s), 8.06-8.10 (2H, m), 8.75 (1H, dd, J=8Hz, 2Hz), 10.61 (1H, s)
IR (ν, c m⁻¹, KBr) : 2220, 1698, 1668, 1640, 1590, 1538, 1474, 1440, 1346, 1160
EI-MS (m/z,%):470 (m+, 39), 360 (24), 296 (40), 205 (100), 176 (100), 151 (42)
m. p.:208-209°C

### Reference Example 54: Trans-N-[2-(4-phenylethynylbenzamido) benzenesulfonyl]-3-hexenamide:

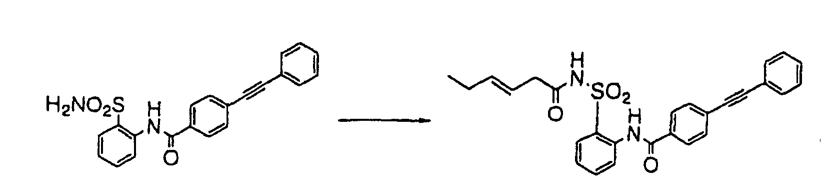

249 mg (1.30 mmol) of tosyl chloride was added to a solution of 149 mg (1.30 mmol) of 3-hexenoic acid and 532 mg (4.35 mmol) of 4-dimethylaminopyridine in anhydrous tetrahydrofuran (10 ml) at 0°C in nitrogen stream. The obtained mixture was stirred for 1 hour and then 300 mg (0.80 mmol) of 4-phenylethynyl-N-(2-sulfamoylphenyl)benzamide prepared in Reference Example 10 was added thereto. The obtained mixture was stirred at room temperature for 1 hour and then the solvent was evaporated under reduced pressure. The residue was dissolved in ethyl acetate and then the obtained solution was washed with water, an aqueous potassium hydrogensulfate solution and saturated aqueous sodium chloride solution in that order. After drying over anhydrous sodium sulfate, the solvent was evaporated under reduced pressure. The residue was purified by the silica gel chromatography to obtain 250 mg (yield: 65.0 %) of the title compound.
¹H-NMR (CDCl₃) δ:0.97 (3H, t, J=7Hz), 2.00-2.10 (2H, m), 2.98 (2H, d, J=7Hz), 5.34-5.42 (1H, m), 5.65-5.72 (1H, m), 7.27 (1H, ddd, J=8Hz, 8Hz, 2Hz), 7.35-7.41 (3H, m), 7.53-7.59 (2H, m), 7.63-7.72 (3H, m), 7.97 (1H, dd, J=8Hz, 2Hz), 8.04 (2H, dd, J=8H z, 2Hz), 8.23 (1H, br-s), 8.73 (1H, dd, J= 8Hz, 2Hz) 10.45 (1H, s)
IR (ν, cm⁻¹, KBr):2220, 1718, 1660, 1602, 1590, 1538, 1444, 1430, 1340, 1128 EI-MS (m/z, %):470 (m+, 71), 360 (19), 296 (3 9), 205 (100), 176 (76), 151 (32)
m. p.: 185.5-186.5°C

### Reference Example 55: 3-Benzyloxy-N-[2[(phenyloxycarbonylamino) sulfonyl]phenyl]benzamide:

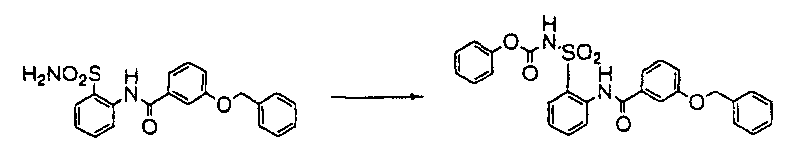

0.36 ml (2.87 mmol) of phenyl chlorocarbonate was added to a solution of 1 g (2.60 mmol) of 3-benzyloxy-N-(2-sulfamoylphenyl) benzamide and 702 mg (5.75 mmol) of 4-dimethylaminopyridine in anhydrous tetrahydrofuran (10 ml) at 0°C in nitrogen stream. The obtained mixture was stirred at room temperature for 1 hour and then the solvent was evaporated under reduced pressure. The residue was dissolved in ethyl acetate and then the obtained solution was washed with water, an aqueous potassium hydrogensulfate solution and saturated aqueous sodium chloride solution in that order. After drying over anhydrous sodium sulfate, the solvent was evaporated under reduced pressure. The residue was recrystallized from acetonitrile to obtain 1.00 g (yield: 77.0 %) of the title compound.
¹H-NMR (CDCl₃) δ:5.10 (2H, s), 6.98-7.01 (2H, m), 7.14-7.21 (2H, m), 7.24-7.32 (4H, m), 7.33-7.46 (5H, m) 7.55 (1H, ddd, J=8H z, 2Hz, 1Hz), 7.60 (1H, dd, J=2Hz, 1Hz), 7.71 (1H, ddd, J=8, 8, 2Hz), 7.80 (1H, br-s), 8.07 (1H, dd, J=8Hz, 2Hz), 8.78 (1H, dd, J = 8 Hz , 1Hz) 10.33 (1H, s)
IR (ν, cm⁻¹, KBr):1762, 1664, 1582, 1534, 1462, 1442, 1360, 1164
FAB-MS (neg:m/z, %):501 ([M-H] + 27), 407 (38), 381 (100)
m. p.:163-164°C

### Reference Example 56: 3-Benzyloxy-N-[2-[[(butylamino)carbonylamino] sulfonyl]phenyl]benzamide:

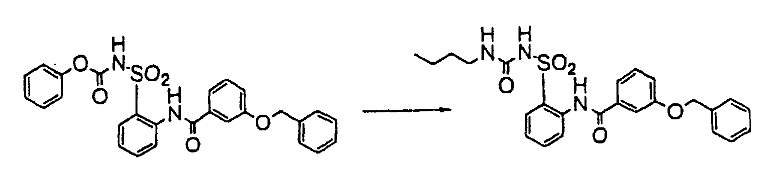

A solution of 200 mg (0.40 mmol) of 3-benzyloxy-N-[2-[(phenyloxycarbonylamino)sulfonyl]phenyl]benzamide prepared in Reference Example 55 and 0.09 ml (0.88 mmol) of butylamine in benzene (10 ml) was heated under reflux for 2 hours. The obtained reaction solution was dissolved in ethyl acetate and then the obtained solution was washed with water, an aqueous potassium hydrogensulfate solution and saturated aqueous sodium chloride solution in that order. After drying over anhydrous sodium sulfate, the solvent was evaporated under reduced pressure. The residue was recrystallized from acetonitrile to obtain 130 mg (yield: 68.0 %) of the title compound.
¹H-NMR (CDCl₃) δ : 0.83 (3H, t, J=7Hz), 1.14-1.29 (2H, m), 1.31-1.37 (2H, m), 2.98 (2 H, d t, J=7, 7Hz), 5.14 (2H, s), 6.11 (1H, br -s), 7.16 (1H, ddd, J=8Hz, 2Hz, 1Hz), 7.23 (1H, ddd, J=8Hz, 8Hz, 1Hz), 7.31-7.42 (5 H, m), 7.45 (2H, dd, J=8, 2Hz), 7.53 (1H, d, J=8Hz), 7.63-7.69 (2H, m), 7.85 (1H, dd, J = 8Hz, 2Hz), 8.38 (1H, br-s), 8.70 (1H, dd, J=8Hz, J=1Hz), 10.13 (1H, s)
IR (ν, cm⁻¹, KBr):1698, 1650, 1580, 1538, 1484, 1450, 1330, 1166
FAB-MS (neg:m/z, %):480 ([M-H] +100), 381 (27)
m. p.:177-178°C

### Reference Example 57: 3-Benzyloxy-N-[2-[[(octylamino)carbonylamino] sulfonyl]phenyl]benzamide:

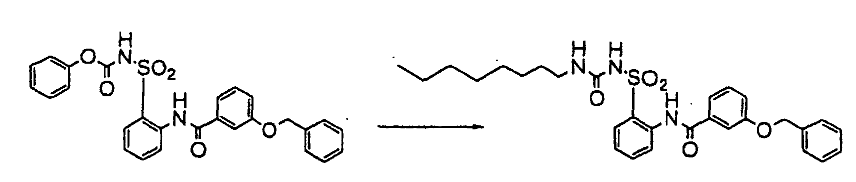

A solution of 200 mg (0.40 mmol) of 3-benzyloxy-N-[2-[(phenyloxycarbonylamino)sulfonyl]phenyl]benzamide prepared in Reference Example 55 and 0.15 ml (0.88 mmol) of octylamine in benzene (10 ml) was heated under reflux for 2 hours. The obtained reaction solution was dissolved in ethyl acetate and then the obtained solution was washed with water, an aqueous potassium hydrogensulfate solution and saturated aqueous sodium chloride solution in that order. After drying over anhydrous sodium sulfate, the solvent was evaporated under reduced pressure. The residue was purified by the silica gel chromatography to obtain 210 mg (yield: 98.0 %) of the title compound.
¹H-NMR(CDCl₃) δ:0.88 (3H, t, J=7Hz), 1.10-1.40 (12H, m), 3.00 (2H, d t, J=7, 7Hz), 5.14 (2H, s), 6.15 (1H, br-s), 7.17 (1H, ddd, J=8Hz, 2Hz, 1Hz), 7.23 (1H, ddd, J=8Hz, 8 Hz, 1Hz), 7.31-7.42 (5H, m), 7.46 (2H, d, J =8Hz), 7.53 (1H, d, J=8Hz), 7.62-7.70 (2H, m), 7.86 (1H, dd, J=8Hz, 2Hz), 8.07 (1H, br -s), 8.71 (1H, d, J=8Hz), 10.11 (1H, s)
IR (ν, cm⁻¹, KBr):1702, 1650, 1580, 1540, 1450, 1356, 1332, 1164
FAB-MS (neg: m/z, %):536 ([M-H]+63), 368 (70), 272 (100)
m. p. : 124-125°C

### Reference Example 58: 4-Phenylethynyl-N-(2-(2-propylthioacetylamino) sulfonylphenyl]benzamide:

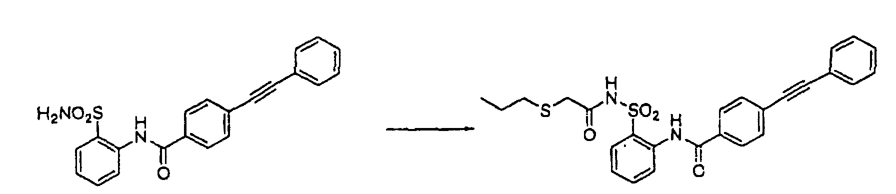

130 mg (1.1 mmol) of 2-propylthioacetic acid was added to a solution of 376 mg (1.0 mmol) of 4-phenylethynyl-N-(2-sulfamoylphenyl)benzamide and 403 mg (3.3 mmol) of 4-dimethylaminopyridine in THF (35 ml). 210 mg (1.1 mmol) of tosyl chloride was slowly added to the obtained mixture. The mixture was stirred at room temperature for 3 hours and then THF was evaporated under reduced pressure. The residue was dissolved in ethyl acetate and then the obtained solution was washed with 1 N aqueous hydrochloric acid solution, water and saturated aqueous sodium chloride solution in that order. After drying over anhydrous sodium sulfate, the solvent was evaporated under reduced pressure. The residue was purified by the silica gel column chromatography to obtain 466 mg (yield: 94.6 %) of the title compound.
¹H-NMR (δ, CDCl₃):0.91 (3H, t, J=7Hz), 1.45-1.5 0 (2H, m), 2.35 (2H, t, J=7Hz), 3.19 (2 H, s), 7.24-7.29 (1H, m), 7.36-7.40 (3H, m), 7.54-7.60 (2H, m), 7.65 (2H, d, J=8Hz), 7.66-7.7 3 (1H, m), 8.01-8.08 (1H, m), 8.04 (2 H, d, J=8Hz 9, 8.76 (1H, dd , J=8, 8Hz), 9.42 (1H, br-s), 10.44 (1H, br-s)
IR (ν, KBr):2216, 1706, 1666, 1588, 1542, 1430, 1340, 1158, 858, 762, 698, 578
FABMS (m/z, %):491 (m-H, 80), 281 (100)
m. p. : 186-188°C

### Reference Example 59: 4-(3-Trifluoromethyl)phenylethynyl-N-(2-sulfamoylphenyl)benzamide:

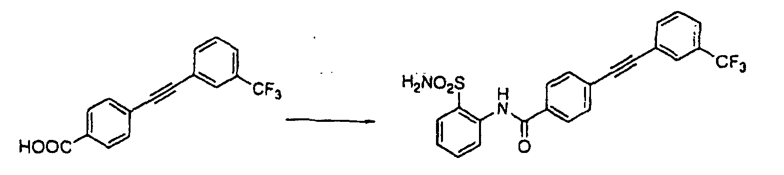

A solution of 2.90 g (10.0 mmol) of 4-(3-trifluoromethyl)phenylethynylbenzoic acid and 2 ml of thionyl chloride in benzene (30 ml) was heated under reflux for 2 hours. The solvent was evaporated under reduced pressure. The residue was dissolved in methylene chloride (30 ml). The obtained solution was added dropwise to a solution of 1.72 g (10.00 mmol) of 2-aminobenzenesulfonamide in pyridine (50 ml) under cooling with ice. The obtained mixture was stirred at room temperature for 18 hours. Methylene chloride was evaporated under reduced pressure. The residue was dissolved in ethyl acetate and then the obtained solution was washed with 1 N aqueous hydrochloric acid solution, water and saturated aqueous sodium chloride solution in that order. After drying over anhydrous sodium sulfate, the solvent was evaporated under reduced pressure. The residue was recrystallized from ethyl acetate - hexane to obtain 3.34 g (yield: 75.3 %) of the title compound.
¹H-NMR (δ, DMSO-d₆):7.34-7.40 (1H, m), 7.65-7.74 (2H, m), 7.76-7.86 (4H, m), 7.91-7.96 (2H, m), 7.98-8.0 2 (3H, m), 8.42-8.46 (1H, m), 10.42 (1H, br-s)

### Reference Example 60: 4-(3-Trifluoromethyl)phenylethynyl-N-(2-hexanoylaminosulfonylphenyl)benzamide:

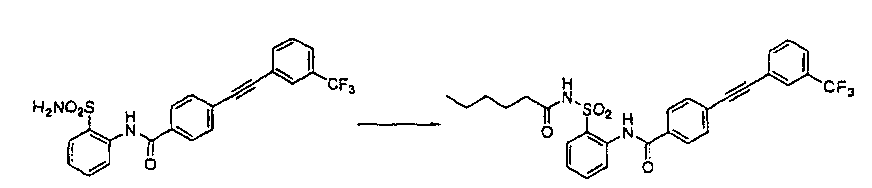

0.16 ml (1.10 mol) of hexanoyl chloride was added to a solution of 444 mg (1.0 mmol) of 4-(3-trifluoromethyl)phenylethynyl-N-(2-sulfamoylphenyl)benzamide and 244 mg (2.0 mmol) of 4-dimethylaminopyridine in THF (35 ml). The obtained mixture was stirred at room temperature for 18 hours and then THF was evaporated under reduced pressure. The residue was dissolved in ethyl acetate and then the obtained solution was washed with 1 N aqueous hydrochloric acid solution, water and saturated aqueous sodium chloride solution in that order. After drying over anhydrous sodium sulfate, the solvent was evaporated under reduced pressure. The residue was purified by the silica gel column chromatography to obtain 504 mg (yield: 92.8 %) of the title compound.
¹H-NMR (δ, DMSO-d₆):0.77 (3H, t, J=7Hz), 1.04-1.22 (4H, m), 1.36-1.45 (2H, m), 2.22 (2 H, t, J=7Hz), 7.39-7.46 (1H, m), 7.69-7.73 (1H, m), 7.73-7.80 (1H, m), 7.80-7.86 (3 H, m), 7.90-8.00 (3H, m), 8.04 (2H, d, J=8H z), 8.36-8.40 (1H, m), 11.48 (1H, s), 12.51 (1H, s)
IR (ν, KBr):3384, 3116, 1718, 1660, 1544, 1510, 1442, 1340, 1170, 1130, 758, 698, 586
EIMS (m/z, %): 542 (m+, 26), 444 (5), 428 (2), 364 (37), 273 (100), 245 (12)
m. p.:194-196°C

### Reference Example 61: 4-(3-Trifluoromethyl)phenylethynyl-N-(2-(5-ketohexanoylamino)sulfonylphenyl)benzamide:

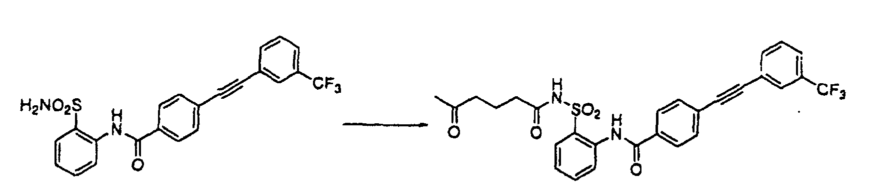

143 mg (1.1 mol) of 5-ketohexanoic acid was added to a solution of 444 mg (1.0 mmol) of 4-(3-trifluoromethyl)phenylethynyl-N-(2-sulfamoylphenyl)benzamide and 403 mg (3.3 mmol) of 4-dimethylaminopyridine in THF (35 ml). 210 mg (1.1 mmol) of tosyl chloride was slowly added to the obtained mixture. The mixture was stirred at room temperature for 3 hours and then THF was evaporated under reduced pressure. The residue was dissolved in ethyl acetate and then the obtained solution was washed with 1 N aqueous hydrochloric acid solution, water and saturated aqueous sodium chloride solution in that order. After drying over anhydrous sodium sulfate, the solvent was evaporated under reduced pressure. The residue was purified by the silica gel column chromatography to obtain 520 mg (yield: 93.4 %) of the title compound.
¹H-NMR (δ, CDCl₃) : 1.75-1.82 (2H, m), 2.09 (3H, s), 2.26 (2H, t, J=7Hz), 2.44 (2H, t, J =7Hz), 7.24-7.29 (1H, m), 7.50 (1H, t, J=7 Hz), 7.60-7.73 (5H, m), 7.82 (1H, d, J=1Hz), 8.00 (1H, dd, J=8, 1Hz), 8.03-8.06 (2H, m), 8.70 (1H, dd, J=7, 1Hz), 9.22 (1H, s), 10.49 (1H, s)
IR (ν, KBr):1716, 1704, 1688, 1588, 1438, 1340, 1296.1126, 760, 696, 590
FABMS (m/z. %): 555 (m-H, 100)
m. p.:169-171°C

### Reference Example 62: 4-(3-Trifluoromethyl)phenylethynyl-N-(2-(2-propyloxyacetylamino)sulfonylphenyl)benzamide:

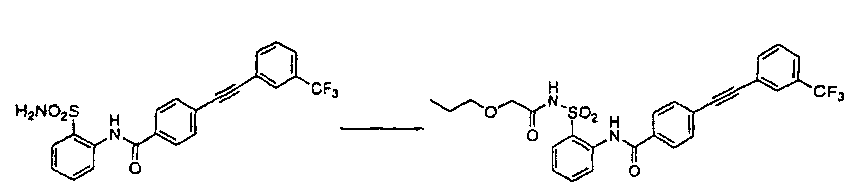

130 mg (1.1 mmol) of 2-propyloxyacetic acid was added to a solution of 444 mg (1.0 mmol) of 4-(3-trifluoromethyl)phenylethynyl-N-(2-sulfamoylphenyl)benzamide and 403 mg (3.3 mmol) of 4-dimethylaminopyridine in THF (35 ml). 210 mg (1.1 mmol) of tosyl chloride was slowly added to the obtained mixture. The mixture was stirred at room temperature for 3 hours and then THF was evaporated under reduced pressure. The residue was dissolved in ethyl acetate and then the obtained solution was washed with 1 N aqueous hydrochloric acid solution, water and saturated aqueous sodium chloride solution in that order. After drying over anhydrous sodium sulfate, the solvent was evaporated under reduced pressure. The residue was purified by the silica gel column chromatography to obtain 496 mg (yield: 91.0 %) of the title compound.
¹H-NMR (δ, CDCl₃): 0.91 (3H, t, J=7Hz), 1.55-1.63 (2H, m), 3.45 (2H, t, J=7Hz), 3.94 (2 H, s), 7.26-7.31 (1H, m), 7.51 (1H, t, J=8H z), 7.60-7.74 (5H, m), 7.82 (1H, s), 8.02-8.08 (3H, m), 8.75 (1H, dd, J=8, 1Hz), 9.03 (1H, s), 10.50 (1H, s)
IR (ν, KBr):3412, 3284, 1724, 1692, 1590, 1342, 1154, 854, 766
FABMS (m/z, %): 543 (m-H, 100)
m. p.:175-177°C

### Reference Example 63: 4-(4-Trifluoromethyl)phenylethynyl-N-(2-sulfamoylphenyl)b enzamide:

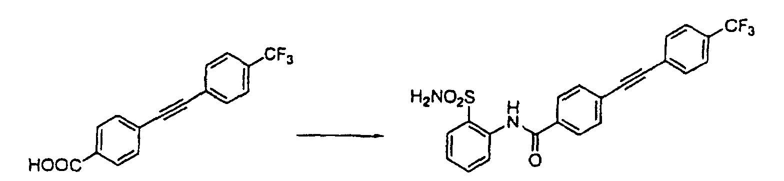

A solution of 2.90 g (10.0 mmol) of 4-(4-trifluoromethyl)phenylethynylbenzoic acid and 2 ml of thionyl chloride in benzene (30 ml) was heated under reflux for 2 hours. The solvent was evaporated under reduced pressure. The residue was dissolved in methylene chloride (30 ml). The obtained solution was added dropwise to a solution of 1.72 g (10.00 mmol) of 2-aminobenzenesulfonamide in pyridine (50 ml) under cooling with ice. The obtained mixture was stirred at room temperature for 18 hours. Methylene chloride was evaporated under reduced pressure. The residue was dissolved in ethyl acetate and then the obtained solution was washed with 1 N aqueous hydrochloric acid solution, water and saturated aqueous sodium chloride solution in that order. After drying over anhydrous sodium sulfate, the solvent was evaporated under reduced pressure. The residue was recrystallized from ethyl acetate - hexane to obtain 3.52 g (yield: 79.3 %) of the title compound.
¹H-NMR (δ, DMSO-d₆) : 7.34-7.40 (1H, m), 7.65-7.70 (1H, m), 7.78 (2H, s), 7.81-7.87 (6 H, m), 7.9 2 (1h, dd, J=8, 1Hz), 7.99 (2H, d, 8Hz), 8.44 (1H, dd, J=8, 1Hz)

### Reference Example 64: 4-(4-Trifluoromethyl)phenylethynyl-N-(2-hexanoylaminosulfonylphenyl)benzamide:

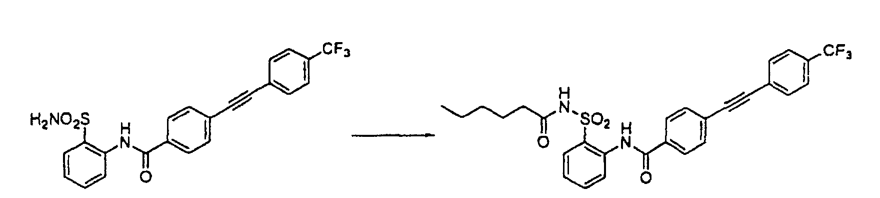

0.16 ml (1.10 mmol) of hexanoyl chloride was added to a solution of 444 g (1.0 mmol) of 4-(4-trifluoromethyl)phenylethynyl-N-(2-sulfamoylphenyl)benzamide and 244 mg (2.0 mmol) of 4-dimethylaminopyridine in THF (35 ml). The mixture was stirred at room temperature for 18 hours and then THF was evaporated under reduced pressure. The residue was dissolved in ethyl acetate and then the obtained solution was washed with 1 N aqueous hydrochloric acid solution, water and saturated aqueous sodium chloride solution in that order. After drying over anhydrous sodium sulfate, the solvent was evaporated under reduced pressure. The residue was purified by the silica gel column chromatography to obtain 513 mg (yield: 94.6 %) of the title compound.
¹H-NMR (δ, DMSO-d₆):0.77 (3H, t, J=7Hz), 1.04-1.22 (4H, m), 1.36-1.45 (2H, m), 2.22 (2 H, t, J=7Hz), 7.40-7.47 (1H, m), 7.74-7.80 (1H, m), 7.80-7.98 (6H, m), 7.96 (1H, dd , J=8, 1Hz), 8.04 (2H, d, J=8Hz), 8.36-8.41 (1H, m), 10.48 (1H, s), 12.52 (1H, s)
IR (ν, KBr):3116, 1700, 1648, 1582, 1534, 1318, 1166, 1134, 844
EIMS (m/z, %):542 (m+, 34), 444 (6), 428 (3), 364 (50), 273 (100), 245 (20)
m. p.:210-212°C

### Reference Example 65: 4-(4-Trifluoromethoxyl)phenylethynyl-N-(2-sulfamoylphenyl)benzamide:

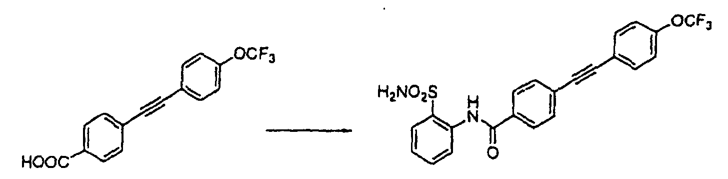

A solution of 3.06 mg (10.0 mmol) of 4-(4-trifluoromethoxyl)phenylethynylbenzoic acid and 2 ml of thionyl chloride in benzene (30 ml) was heated under reflux for 2 hours. The solvent was evaporated under reduced pressure. The residue was dissolved in methylene chloride (30 ml). The obtained solution was added dropwise to a solution of 1.72 g (10.00 mmol) of 2-aminobenzenesulfonamide in pyridine (50 ml) under cooling with ice. The obtained mixture was stirred at room temperature for 18 hours. Methylene chloride was evaporated under reduced pressure. The residue was dissolved in ethyl acetate and then the obtained solution was washed with 1 N aqueous hydrochloric acid solution, water and saturated aqueous sodium chloride solution in that order. After drying over anhydrous sodium sulfate, the solvent was evaporated under reduced pressure. The residue was recrystallized from ethyl acetate - hexane to obtain 3.32 g (yield: 72.1 %) of the title compound.
¹H-NMR (δ, CDCl₃): 4.85-5.10 (2H, br-s), 7.20-7.24 (2H, m), 7.25-7.30 (1H, m), 7.58-7.62 (3H, m), 7.64 (2H, d, J = 8 Hz), 7.95 (2H, d, J=8Hz), 7.98 (1H, dd, J=8, 1Hz), 8.56 (1 H, dd, J=8, 1Hz), 10.13 (1H, s)

### Reference Example 66: 4-(4-Trifluoromethoxyl)phenylethynyl-N-(2-hexanoylaminosulfonylphenyl)benzamide:

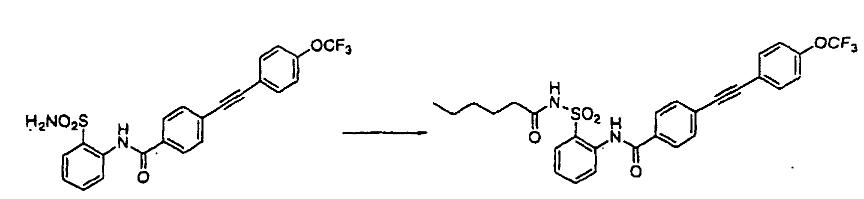

0.16 ml (1.10 mol) of hexanoyl chloride was added to a solution of 460 g (1.0 mmol) of 4-(4-trifluoromethoxyl)phenylethynyl-N-(2-sulfamoylphenyl)benzamide and 244 mg (2.0 mmol) of 4-dimethylaminopyridine in THF (35 ml). The mixture was stirred at room temperature for 18 hours and then THF was evaporated under reduced pressure. The residue was dissolved in ethyl acetate and then the obtained solution was washed with 1 N aqueous hydrochloric acid solution, water and saturated aqueous sodium chloride solution in that order. After drying over anhydrous sodium sulfate, the solvent was evaporated under reduced pressure. The residue was purified by the silica gel column chromatography to obtain 517 mg (yield: 95.7 %) of the title compound.
¹H-NMR (δ, DMSO-d₆):0.77 (3H, t, J=7Hz), 1.03-1.22 (4H, m), 1.36-1.44 (2H, m), 2.22 (2 H, t, J=7Hz), 7.40-7.46 (1H, m), 7.47 (2H, d, J=8Hz), 7.75 (2H, d, J=8Hz), 7.75 (2H, d, J=8Hz), 7.80 (2H, d, J=8Hz), 7.95 (1H, dd, J=8, 1Hz), 8.03 (2H, d, J=8Hz), 8.38-8.41 (1H, m), 10.47 (1H, s), 12.51 (1H, s)
IR (ν,KBr):3112, 1700, 1650, 1582, 1516, 1250, 1166, 858
EIMS (m/z, %):558 (m+, 22), 460 (3), 380 (1 8), 289 (100), 261 (17)
m. p.:197-199°C

### Reference Example 67: 4-(4-Trifluoromethoxyl)phenylethynyl-N-(2-(5-ketohexanoylamino)sulfonylphenyl)benzamide:

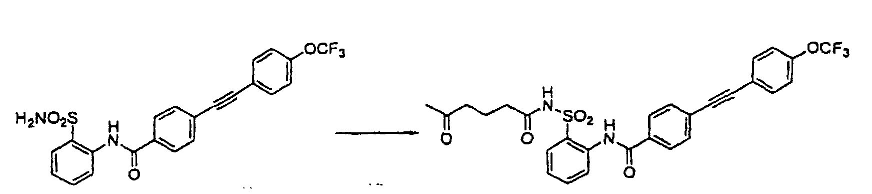

143 mg (1.10 mmol) of 5-ketohexanoic acid was added to a solution of 460 mg (1.0 mmol) of 4-(4-trifluoromethoxyl)phenylethynyl-N-(2-sulfamoylphenyl)benzamide and 403 mg (3.3 mmol) of 4-dimethylaminopyridine in THF (35 ml). Then 210 mg (1.1 mmol) of tosyl chloride was slowly added thereto. The mixture was stirred at room temperature for 3 hours and then THF was evaporated under reduced pressure. The residue was dissolved in ethyl acetate and then the obtained solution was washed with 1 N aqueous hydrochloric acid solution, water and saturated aqueous sodium chloride solution in that order. After drying over anhydrous sodium sulfate, the solvent was evaporated under reduced pressure. The residue was purified by the silica gel column chromatography to obtain 538 mg (yield: 94.0 %) of the title compound.
¹H-NMR (δ, CDCl₃):1.75-1.82 (2H, m), 2.09 (3H, s), 2.27 (2H, t, J=7Hz), 2.46 (2H, t, J =7Hz), 7.21-7.30 (5H, m), 7.56-7.71(3H, m), 8.01-8.06 (3H, m), 8.72 (1H, dd, J=8, 1 Hz), 9.07 (1H, s), 10.46 (1H, s)
IR (ν, KBr) : 3320, 1736, 1716, 1652, 1582, 1538, 1516, 1444, 1250, 1136, 856, 764, 576
FABMS (m/z, %):571 (m-H, 18), 459 (100)
m. p.: 204-205°C

### Reference Example 68: 4-(4-Fluoro)phenylethynyl-N-(2-sulfamoylphenyl)benzamide:

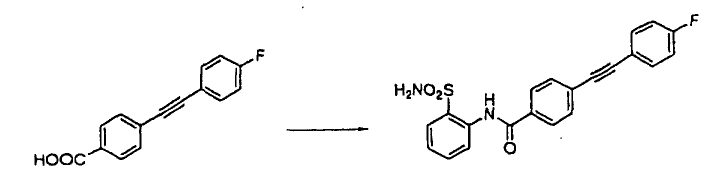

A solution of 2.40 g (10.0 mmol) of 4-(4-fluoro)phenylethynylbenzoic acid and 2 ml of thionyl chloride in benzene (30 ml) was heated under reflux for 2 hours. Then the solvent was evaporated under reduced pressure. The residue was dissolved in methylene chloride (30 ml). The obtained solution was added dropwise to a solution of 1.72 g (10.00 mmol) of 2-aminobenzenesulfonamide in pyridine (50 ml) under cooling with ice. The mixture was stirred at room temperature for 18 hours and then methylene chloride was evaporated under reduced pressure. The residue was dissolved in ethyl acetate and then the obtained solution was washed with 1 N aqueous hydrochloric acid solution, water and saturated aqueous sodium chloride solution in that order. After drying over anhydrous sodium sulfate, the solvent was evaporated under reduced pressure. The residue was recrystallized from ethyl acetate - hexane to obtain 3.08 mg (yield: 78.2 %) of the title compound.
¹H-NMR (δ, DMSO-d₆):7.29-7.38 (3H, m), 7.65-7.70 (3H,m), 7.76-7.78 (4H, m), 7.92 (1 H, dd, J=8Hz, 1Hz), 7.91 (1H, dd, J=8.1Hz), 7.96 (2H, d, J=8Hz), 8.44 (1H, d, J=7Hz), 10.42 (1H, br-s)

### Reference Example 69: 4-(4-Fluoro)phenylethynyl-N-(2-hexanoylaminosulfonylphenyl)benzamide:

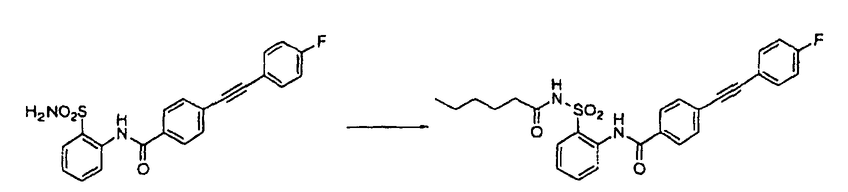

0.16 ml (1.10 mol) of hexanoyl chloride was added to a solution of 394 g (1.0 mmol) of 4-(4-fluoro)phenylethynyl-N-(2-sulfamoylphenyl)benzamide and 244 mg (2.0 mmol) of 4-dimethylaminopyridine in THF (35 ml). The mixture was stirred at room temperature for 18 hours and then THF was evaporated under reduced pressure. The residue was dissolved in ethyl acetate and then the obtained solution was washed with 1 N aqueous hydrochloric acid solution, water and saturated aqueous sodium chloride solution in that order. After drying over anhydrous sodium sulfate, the solvent was evaporated under reduced pressure. The residue was purified by the silica gel column chromatography to obtain 471 mg (yield: 95.7 %) of the title compound.
¹H-NMR (δ, DMSO-d₆): 0.77 (3H, t, J=7Hz), 1.08-1.12 (2H, m), 1.12-1.20 (2H, m), 1.38-1.42 (2H, m), 2.22 (2H, t, J=7Hz), 7.30-7.35 (2H, m), 7.42 (1H, dt , J = 7,1Hz), 7.66-7.71 (1H, m), 7.74-7.90 (3H, m), 7.95 (1H, dd , J=8, 1Hz), 8.01 (2H, d, J=8Hz), 8.38 (2H, d, J = 8 Hz), 10.47 (1H, s), 12.52 (1H, br-s)
IR (ν, KBr):3372, 1706, 1658, 1588, 1540, 1516, 1320, 834, 766
EIMS (m/z, %) : 492 (m+, 26), 394 (6), 378 (3), 314 (25), 223 (100), 194 (15)
m. p.:183-186°C

### Reference Example 70: 4-(4-Fluoro)phenylethynyl-N-(2-(5-ketohexanoylamino)sulfonylphenyl)benzamide:

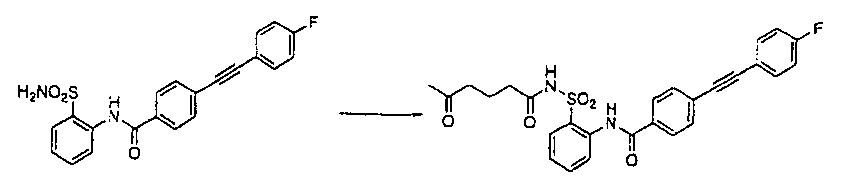

143 mg (1.1 mmol) of 5-ketohexanoic acid was added to a solution of 394 g (1.0 mmol) of 4-(4-fluoro)phenylethynyl-N-(2-sulfamoylphenyl)benzamide and 403 mg (3.3 mmol) of 4-dimethylaminopyridine in THF (35 ml). Then 210 mg (1.1 mmol) of tosyl chloride was slowly added thereto. The mixture was stirred at room temperature for 3 hours and then THF was evaporated under reduced pressure. The residue was dissolved in ethyl acetate and then the obtained solution was washed with 1 N aqueous hydrochloric acid solution, water and saturated aqueous sodium chloride solution in that order. After drying over anhydrous sodium sulfate, the solvent was evaporated under reduced pressure. The residue was purified by the silica gel chromatography to obtain 472 mg (yield: 93.1 %) of the title compound.
¹H-NMR (δ, DMSO-d₆): 1.55-1.63 (2H, m), 2.00 (3H, s), 2.23 (2H, t, J=7Hz), 2.31 (2H, t, J=7Hz), 7.32 (2H, d, J=8Hz), 7.43 (1H, t, J =7Hz), 7.66-7.70 (2H, m), 7.76-7.78 (3H, m), 7.96 (1H, d, J=7Hz), 8.01 (2H, d, J=8Hz), 8.35 (1H, d, J=8Hz), 10.44 (1H, s), 12.52 (1H, s)
IR (ν, KBr):1722, 1698, 1680, 1514, 1294, 854, 758, 584
FABMS (m/z, %) : 505 (m-H, 90), 393 (100)
m. p.:179-181°C

### Reference Example 71: 4-(3-Fluoro)phenylethynyl-N-(2-sulfamoylphenyl)benzamide:

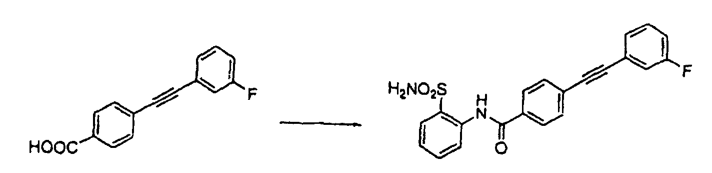

A solution of 2.40 g (10.0 mmol) of 4-(3-fluoro)phenylethynylbenzoic acid and 2 ml of thionyl chloride in benzene (30 ml) was heated under reflux for 2 hours. Then the solvent was evaporated under reduced pressure. The residue was dissolved in methylene chloride (30 ml). The obtained solution was added dropwise to a solution of 1.72 g (10.00 mmol) of 2-aminobenzenesulfonamide in pyridine (50 ml) under cooling with ice. The mixture was stirred at room temperature for 18 hours and then methylene chloride was evaporated under reduced pressure. The residue was dissolved in ethyl acetate and then the obtained solution was washed with 1 N aqueous hydrochloric acid solution, water and saturated aqueous sodium chloride solution in that order. After drying over anhydrous sodium sulfate, the solvent was evaporated under reduced pressure. The residue was recrystallized from ethyl acetate - hexane to obtain 3.06 g (yield: 77.7 %) of the title compound.
¹H-NMR (δ, DMSO-d₆): 7.30-7.40 (2H, m), 7.45-7.54 (3H, m), 7.65-7.70 (1H, m), 7.76-7.82 (4H, m), 7.93 (1H, dd, J=8, 1Hz), 7.79 (2H, d, J=8Hz), 8.44 (1H, dd, J=8, 1Hz)

### Reference Example 72: 4-(3-Fluoro)phenylethynyl-N-(2-hexanoylaminosulfonylphenyl)benzamide:

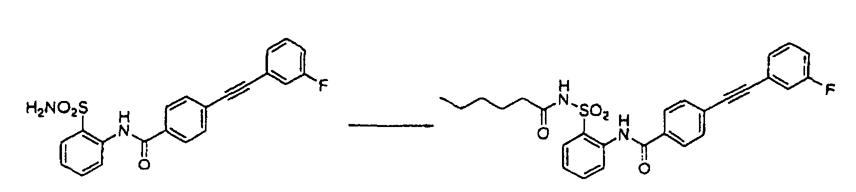

0.16 ml (1.10 mol) of hexanoyl chloride was added to a solution of 394 g (1.0 mmol) of 4-(3-fluoro)phenylethynyl-N-(2-sulfamoylphenyl)benzamide and 244 mg (2.0 mmol) of 4-dimethylaminopyridine in THF (35 ml). The mixture was stirred at room temperature for 18 hours and then THF was evaporated under reduced pressure. The residue was dissolved in ethyl acetate and then the obtained solution was washed with 1 N aqueous hydrochloric acid solution, water and saturated aqueous sodium chloride solution in that order. After drying over anhydrous sodium sulfate, the solvent was evaporated under reduced pressure. The residue was purified by the silica gel chromatography to obtain 466 mg (yield: 94.6 %) of the title compound.
¹H-NMR (δ, DMSO-d₆) : 0.77 (3H, t, J= 7Hz), 1.03-1.22 (4H, m), 1.35-1.45 (2H, m), 2.22 (2 H, t, J=7Hz), 7.30-7.36 (1H, m), 7.40-7.55 (4H, m), 7.74-7.82 (3H, m), 7.95 (1H, dd , J=8, 1Hz), 8.03 (2H, d, J=8Hz), 8.36-8.41 (1H, m), 10.47 (1H, s), 12.51 (1H, s)
IR (ν, KBr):1706, 1658, 1588, 1540, 1342, 1142, 862, 768, 584
EIMS (m/z, %) : 492 (m+, 21), 394 (4), 378 (2), 314 (22), 223 (100), 194 (20)
m.p.:183-186°C

### Example 1

Effect of accelerating the glucose uptake in cultured skeletal muscle cells: Method: Rat skeletal muscle cell strain "L6 cells" in a confluent state was cultured in DME medium containing 2 % of fetal calf serum for one week to differentiate into myotubes. The medium was replaced with the DME medium containing a compound synthesized in each of the above Reference Examples. After culturing overnight followed by the thorough washing with HEPES buffer solution, an HEPES buffer solution containing 37 kBq/ml of 10 µM [3H]-2-deoxyglucose (2DG) was added thereto. After the culture at 37°C for 10 minutes, the radioactivity of 2DG transmigrated into the cells was determined. The effect (%) of each compound on 2DG uptake was determined as compared with that in the control group. The results are shown in Table 1.

### Example 2

### Hypoglycemic effect on non-insulin-dependent diabetes model mice (KK mice)

Method: A compound (30 mg/kg) was administered to non-insulin-dependent diabetes model mice (8 weeks old) twice a day for one week. After final administration, the mice were fasted overnight and then the blood glucose concentration of each mouse was determined. The hypoglycemic effect of the compound was calculated on the basis of the blood glucose level (100) in the control group. The results are shown in Table 2.

**Table 1**

| Ref. Ex. | Effect of accelerating the glucose uptake (%) at 10 µM |
|---|---|
| 26 | 134 |
| 30 | 131 |
| 34 | 145 |
| 41 | 230 |
| 49 | 212 |
| 51 | 225 |
| 54 | 118 |
| 60 | 246 |
| 64 | 204 |
| 66 | 94 |
| 69 | 240 |
| 72 | 279 |

**Table 2**

| Ref. Ex. | Hypoglycemic effect (%) |
|---|---|
| 49 | 10.3 |
| 60 | 51.1 |
| 61 | 65.7 |
| 62 | 60.9 |
| 64 | 34.5 |
| 66 | 28.2 |
| 67 | 40.2 |
| 69 | 27.7 |
| 70 | 30.5 |
| 58 | 22.7 |
| 72 | 9.2 |

The present invention provides a hypoglycemic agent and also an ACC activity-inhibiting hypoglycemic agent. They are usable for preventing and treating diabetes. The hypoglycemic agent that also has ACC activity-inhibiting effect is also capable of controlling the accumulation of visceral fats. The medical effects of the hypoglycemic agent as a new medicine are great.

## Claims

1. A hypoglycemic agent containing an acylsulfonamide derivative(s) of the following general formula (I) as the active ingredient(s): 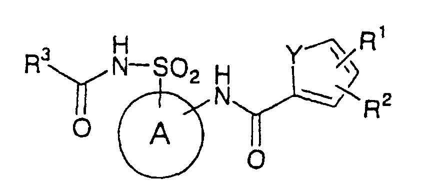 wherein R¹ represents a substituted or unsubstituted C₁ to C₁₂ alkyl group, a substituted or unsubstituted C₂ to C₁₂ alkenyl group, a substituted or unsubstituted C₂ to C₁₂ alkynyl group or a substituted or unsubstituted C₁ to C₁₂ alkoxyl group, R² represents hydrogen atom, hydroxyl group, mercapto group, a substituted or unsubstituted C₁ to C₆ alkoxyl group, a substituted or unsubstituted C₁ to C₆ alkylthio group, nitro group, a halogen atom, trichloromethyl group, trifluoromethyl group or cyano group, R³ represents a substituted or unsubstituted C₁ to C₂₀ alkyl group, a substituted or unsubstituted C₂ to C₂₀ alkenyl group, a substituted or unsubstituted C₂ to C₂₀ alkynyl group, a substituted or unsubstituted aromatic hydrocarbon group, a substituted or unsubstituted aromatic heterocyclic group, a substituted amino group, a substituted or unsubstituted C₁ to C₂₀ alkoxyl group, a substituted or unsubstituted C₂ to C₂₀ alkenyloxyl group, a substituted or unsubstituted C₂ to C₂₀ alkynyloxyl group or a group of the formula: R⁴O-, wherein R⁴ represents a substituted or unsubstituted aromatic hydrocarbon group or a substituted or unsubstituted aromatic heterocyclic group, Y represents a group of the formula: -CH=CH-, -N=CH- or -CH=N-, sulfur atom or oxygen atom, and ring A represents a substituted or unsubstituted aromatic hydrocarbon group, a substituted or unsubstituted aromatic heterocyclic group or a substituted or unsubstituted cycloalkyl group.

2. The hypoglycemic agent according to claim 1 wherein R³ represents a substituted or unsubstituted C₃ to C₈ alkyl group, a substituted or unsubstituted C₄ to C₈ alkenyl group, a substituted or unsubstituted C₄ to C₈ alkynyl group, a substituted or unsubstituted phenyl group, a substituted or unsubstituted naphthyl group, a substituted or unsubstituted C₃ to C₉ alkoxyl group, a substituted or unsubstituted C₄ to C₈ alkenyloxyl group or a substituted or unsubstituted C₄ to C₈ alkynyloxyl group.

3. The hypoglycemic agent according to claim 1 or 2 wherein ring A is a substituted or unsubstituted phenyl group.

4. The hypoglycemic agent according to any of claims 1 to 3 wherein ring A is an aromatic hydrocarbon group having substitution sites at the 1,2-position, an aromatic heterocyclic group having substitution sites at the 1,2-position or a cycloalkyl group having substitution sites at the 1,1-position.

5. The hypoglycemic agent according to any of claims 1 to 4 wherein R¹ represents a C₁ to C₄ alkyl group having a substituted or unsubstituted aromatic hydrocarbon group or a substituted or unsubstituted aromatic heterocyclic group as the substituent, a C₂ to C₄ alkenyl group having a substituted or unsubstituted aromatic hydrocarbon group or a substituted or unsubstituted aromatic heterocyclic group as the substituent, a C₂ to C₄ alkynyl group having a substituted or unsubstituted aromatic hydrocarbon group or a substituted or unsubstituted aromatic heterocyclic group as the substituent, or C₁ to C₄ alkoxyl group having a substituted or unsubstituted aromatic hydrocarbon group or a substituted or unsubstituted aromatic heterocyclic group as the substituent.

6. The hypoglycemic agent according to claim 5 wherein R¹ represents a substituted ethynyl group.

7. The hypoglycemic agent according to claim 6 wherein the substituent of ethynyl group R¹ is phenyl group substituted with one or more fluorine atoms, trifluoromethyl groups or trifluoromethoxyl groups.

8. The hypoglycemic agent according to claim 7 wherein the substituent of ethynyl group R¹ is a group of the following formula:

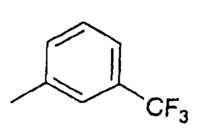

9. The hypoglycemic agent according to claim 7 wherein the substituent of ethynyl group R¹ is a group of the following formula:

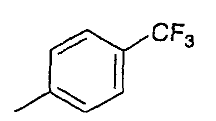

10. The hypoglycemic agent according to claim 7 wherein the substituent of ethynyl group R¹ is a group of the following formula:

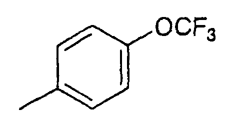

11. A hypoglycemic agent containing a compound having an activity of inhibiting acetyl CoA carboxylase as an active ingredient.

12. The hypoglycemic agent according to claim 11 wherein the active ingredient is an acylsulfonamide derivative of the following general formula: 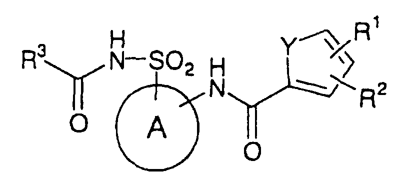 wherein R¹ represents a substituted or unsubstituted C₁ to C₁₂ alkyl group, a substituted or unsubstituted C₂ to C₁₂ alkenyl group, a substituted or unsubstituted C₂ to C₁₂ alkynyl group or a substituted or unsubstituted C₁ to C₁₂ alkoxyl group, R² represents hydrogen atom, hydroxyl group, mercapto group, a substituted or unsubstituted C₁ to C₆ alkoxyl group, a substituted or unsubstituted C₁ to C₆ alkylthio group, nitro group, a halogen atom, trichloromethyl group, trifluoromethyl group or cyano group, R³ represents a substituted or unsubstituted C₁ to C₂₀ alkyl group, a substituted or unsubstituted C₂ to C₂₀ alkenyl group, a substituted or unsubstituted C₂ to C₂₀ alkynyl group, a substituted or unsubstituted aromatic hydrocarbon group, a substituted or unsubstituted aromatic heterocyclic group, a substituted amino group, a substituted or unsubstituted C₁ to C₂₀ alkoxyl group, a substituted or unsubstituted C₂ to C₂₀ alkenyloxyl group, a substituted or unsubstituted C₂ to C₂₀ alkynyloxyl group or a group of the formula: R⁴O-, wherein R⁴ represents a substituted or unsubstituted aromatic hydrocarbon group or a substituted or unsubstituted aromatic heterocyclic group, Y represents a group of the formula: -CH=CH-, -N=CH- or -CH=N-, sulfur atom or oxygen atom, and ring A represents a substituted or unsubstituted aromatic hydrocarbon group, substituted or unsubstituted aromatic heterocyclic group or a substituted or unsubstituted cycloalkyl group.

13. The hypoglycemic agent according to claim 12 wherein R³ represents a substituted or unsubstituted C₃ to C₈ alkyl group, a substituted or unsubstituted C₄ to C₈ alkenyl group, a substituted or unsubstituted C₄ to C₈ alkynyl group, a substituted or unsubstituted phenyl group, a substituted or unsubstituted naphthyl group, a substituted or unsubstituted C₃ to C₉ alkoxyl group, a substituted or unsubstituted C₄ to C₈ alkenyloxyl group or a substituted or unsubstituted C₄ to C₈ alkynyloxyl group.

14. The hypoglycemic agent according to claim 12 or 13 wherein ring A is a substituted or unsubstituted phenyl group.

15. The hypoglycemic agent according to any of claims 12 to 14 wherein ring A is an aromatic hydrocarbon group having substitution sites at the 1,2-position, an aromatic heterocyclic group having substitution sites at the 1,2-position or a cycloalkyl group having substitution sites at the 1,1-position.

16. The hypoglycemic agent according to any of claims 12 to 15 wherein R¹ represents a C₁ to C₄ alkyl group having a substituted or unsubstituted aromatic hydrocarbon group or a substituted or unsubstituted aromatic heterocyclic group as the substituent, a C₂ to C₄ alkenyl group having a substituted or unsubstituted aromatic hydrocarbon group or a substituted or unsubstituted aromatic heterocyclic group as the substituent, a C₂ to C₄ alkynyl group having a substituted or unsubstituted aromatic hydrocarbon group or a substituted or unsubstituted aromatic heterocyclic group as the substituent, or C₁ to C₄ alkoxyl group having a substituted or unsubstituted aromatic hydrocarbon group or a substituted or unsubstituted aromatic heterocyclic group as the substituent.

17. The hypoglycemic agent according to claim 16 wherein R¹ represents a substituted ethynyl group.

18. The hypoglycemic agent according to claim 17 wherein the substituent of ethynyl group R¹ is phenyl group substituted with one or more fluorine atoms, trifluoromethyl groups or trifluoromethoxyl groups.

19. The hypoglycemic agent according to claim 18 wherein the substituent of ethynyl group R¹ is a group of the following formula:

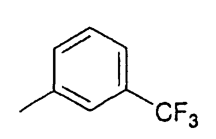

20. The hypoglycemic agent according to claim 18 wherein the substituent of ethynyl group R¹ is a group of the following formula:

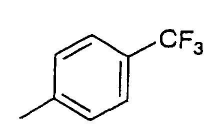

21. The hypoglycemic agent according to claim 18 wherein the substituent of ethynyl group R¹ is a group of the following formula:

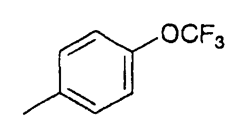

22. An agent for improving insulin resistance, which contains a compound having an activity of inhibiting acetyl CoA carboxylase as an active ingredient.

23. The agent for improving insulin resistance according to claim 12, which contains an acylsulfonamide derivative of general formula (I) of any of claims 1 to 10.

24. An acetyl CoA carboxylase inhibitor containing an acylsulfonamide derivative of the general formula (I) according to any of claims 1 to 10 as an active ingredient.
